(19) Europäisches Patentamt
European Patent Office
Office européen des brevets

(11) **EP 4 775 218 A1**

(12) **EUROPEAN PATENT APPLICATION**
published in accordance with Art. 153(4) EPC

(43) Date of publication:
**15.07.2026  Bulletin 2026/29**

(21) Application number: **24861989.2**

(22) Date of filing: **04.09.2024**

(51) International Patent Classification (IPC):
**A61K 38/48** (2006.01)    **A61P 35/00** (2006.01)

(52) Cooperative Patent Classification (CPC):
**A61K 38/48; A61P 35/00**

(86) International application number:
**PCT/CN2024/116797**

(87) International publication number:
**WO 2025/051146 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **07.09.2023  CN 202311153380**

(71) Applicant: **Academy of Military Medical Sciences
Beijing 100850 (CN)**

(72) Inventors:
• **ZHONG, Wu
  Beijing 100850 (CN)**
• **CAO, Ruiyuan
  Beijing 100850 (CN)**

• **TAO, Huimin
  Beijing 100850 (CN)**
• **YANG, Xiaotong
  Beijing 100850 (CN)**
• **LI, Wei
  Beijing 100850 (CN)**
• **LI, Song
  Beijing 100850 (CN)**

(74) Representative: **Lavoix
Bayerstraße 83
80335 München (DE)**

Remarks:
The complete document including Reference Table(s) and the Sequence Listing(s) can be downloaded from the EPO website

(54) **USE OF CORONAVIRUS 3CL PROTEASE FOR PREVENTING OR TREATING TUMORS**

(57)    A use of a coronavirus 3CL protease or a nucleic acid molecule encoding the coronavirus 3CL protease in preparation of drugs for preventing and/or treating tumors.

## Description

The present application is based on and claims the benefit of priority from Chinese Application No. 202311153380.8, filed on Sep. 07, 2023, the disclosures of which are incorporated herein by reference in its entirety.

## Technical Field

The present application belongs to the field of medical biotechnology, and specifically relates to the use of a 3CL protease of coronavirus or a nucleic acid molecule encoding the same in the preparation of a medicament for the prevention and/or treatment of tumors.

## Background Art

Cancer is a disease characterized by uncontrolled growth of cells induced by genetic mutation or dysregulation, and exhibits strong spatiotemporal heterogeneity depending on occurrence site. The application of a cancer therapeutic drug requires not only overcoming this heterogeneity, but also achieving the goal of selectively killing cancer cells with minimal toxicity to normal cells. In the research and development of anti-cancer drugs, how to reduce the toxicity of current clinical medications to the body while maintaining the broad-spectrum anti-cancer activity of the drugs is always a challenging problem. For different types of cancer, including solid and non-solid tumors, current clinical treatment regimens typically include one or a combination of surgery, chemotherapy, radiotherapy, bone marrow/stem cell transplantation, hormonal therapy, targeted therapy, and immunotherapy. Although the seven types of mainstream anti-tumor therapies can achieve the purpose of eliminating tumors to a certain extent, patients, upon administration, may develop drug resistance and face a risk of recurrence risk, and may experience pain or suffer irreversible physical damage during multiple rounds of treatment. Novel and effective anti-tumor therapeutic methods are urgently needed to be developed and applied.

Gene therapy and genetic vaccination belong to anti-tumor strategies which are rapidly developed in recent years, offering specific and individualized options for the treatment and prevention of various tumors. Currently, there are only a few gene therapies approved for oncotherapy in the world, such as Genticine for the treatment of head and neck squamous cell carcinoma, Imlygic for the treatment of melanoma lesions that cannot be completely resected by surgery, Kymriah for the treatment of patients aged 3 to 25 years with relapsed or refractory acute lymphoblastic leukemia, and Yescatta for the treatment of adult patients with relapsed or refractory diffuse large B-cell lymphoma after two or more lines of systemic therapy and adult patients with primary mediastinal large B-cell lymphoma. The gene therapy represented by these four therapies exerts anti-tumor effects by introducing suicide genes, immune regulation genes into cells by using viruses, modified immune cells or other delivery vectors, and inserting them into existing genome, thereby eliminating the effects of harmful mutations in genes or stimulating the anti-tumor immune response of patients. In addition to this method of introducing exogenous genes, delivery vectors may be also used to mediate the entry of oligonucleotides into tumor cells, thereby inhibiting or interfering with endogenous genes, to achieve therapeutic purpose.

In addition, researchers have developed a variety of DNA vaccines or RNA vaccines encoding tumor antigens for the prevention and treatment of tumors, which belong to the field of genetic vaccine. Relevant studies have shown that messenger RNA vaccine has been a promising strategy for cancer immunotherapy. After the vaccination with a naked vaccine or a drug-loaded mRNA vaccine, tumor antigens will be expressed in antigen presenting cells (APCs), thereby promoting APC activation and innate/adaptive immunity. The mRNA cancer vaccine has advantages such as high efficiency, safety, great development potential, low production cost, surpassing other conventional vaccine platforms. Tumor vaccine, as a means of immunotherapy, highly depends on the body's own immune system, while the immune system of cancer patients is often suppressed with compromised defense functions, which imposes certain limitations on the application of tumor vaccines.

The mRNA therapy in gene therapy has been explored as one of the safest and most effective approaches for tumor treatment. The mRNA consists of four nucleotides, abbreviated as A, U, C and G. Cells decode the sequence of these nucleotides arrangements, if the information encoded by mRNA cannot read, the corresponding protein with specific structure and function cannot be translated, the mRNA will be finally metabolized and degraded by cells without producing relevant biological effect. If an opposite situation occurs, the body will only produce the proteins it needs at the right time and in the right place. Therefore, the mRNA-based drug therapy is considered to be an accurate and safe anti-tumor strategy.

Thus, there is still a great medical demand for anti-tumor mRNA drugs to improve patient's survival rate and bring greater therapeutic benefits to patients themselves, patient's family and society.

**Summary of the present application**

The inventors of the present application have surprisingly found that the 3CL protease of coronavirus (for example, SARS-CoV-2) has a broad-spectrum and significant tumor cell killing capacity. Based on this finding, the inventors of the present application have developed a method for the prevention and/or treatment of a tumor based on the 3CL protease or a nucleic acid molecule encoding the 3CL protease. The therapeutic drug (for example, a nucleic acid drug), provided by the present application, which is based on the 3CL protease or the nucleic acid molecule encoding the 3CL protease, is expected to expand the benefit range of cancer patients, improve the survival rate of patients and bring more therapeutic benefits to patients themselves, patient's family and society.

A first aspect of the present application provides a use of a 3CL protease of coronavirus or a nucleic acid molecule encoding the 3CL protease of coronavirus in the preparation of a medicament for the prevention and/or treatment of a tumor in a subject.

In certain embodiments, the coronavirus is SARS-CoV, MERS-CoV or SARS-CoV-2.

In certain embodiments, the coronavirus is SARS-CoV-2. In certain embodiments, the SARS-CoV-2 comprises not only the original strain of SARS-CoV-2 but also the variants thereof, such as Alpha, Beta, Gamma, Delta and Omicron variants.

It is easy for those skilled in the art to understand that the "3CL protease" described herein may be derived directly from the coronavirus (e.g., SARS-CoV, MERS-CoV, or SARS-CoV-2), or alternatively, obtained by artificial modification (e.g., introduction of mutation or modification) of the 3CL protease derived directly from the coronavirus, provided that it retains the biological activity (e.g., tumor cell killing activity or oncolytic activity) of the 3CL protease from which it is derived. In addition, the 3CL protease described herein may be a full-length 3CL protease or an active fragment thereof (e.g., an active segment having tumor cell killing activity or oncolytic activity).

In certain embodiments, the 3CL protease is derived from SARS-CoV (e.g., SARS coronavirus strain BJ01 (Genbank: AY278488.2), SARS coronavirus stain BJ03 (Genbank: AY278490.3), and SARS coronavirus stain Tor2 (Genbank: AY274119.3)). In certain embodiments, the 3CL protease is as set forth in any one of Uniprot: P0C6U8, P0C6X7, and B8Q8V7.

In certain embodiments, the 3CL protease is derived from MERS-CoV (e.g., Middle East respiratory syndrome coronavirus isolate Bisha_1_2012 (Genbank: KF600620.1), Middle East respiratory syndrome-related coronavirus isolate MERS-CoV/dromedary camel/Egypt/NC282/2015 (Genbank: OP654178.1), and Middle East respiratory syndrome coronavirus isolate Riyadh_2_2012 (Genbank: KF600652.1)). In certain embodiments, the 3CL protease is as set forth in any one of Uniprot: K9N638, K9N7C7, and A0A023W112.

In certain embodiments, the 3CL protease is derived from SARS-CoV-2 (e.g., Severe acute respiratory syndrome coronavirus 2 isolate Wuhan-Hu-1 (Genbank: NC_045512.2), Severe acute respiratory syndrome coronavirus 2 isolate SARS-CoV-2/human/MEX/CMX_2019/2022 (Genbank: OR157051.1), and Severe acute respiratory syndrome coronavirus 2 isolate SARS-CoV-2/human/LBY/BTRC Libya SARS-CoV-2 WGS-33/2021 (Genbank: MZ054889.1)). In certain embodiments, the 3CL protease is as set forth in any one of Uniprot: P0DTC1, P0DTD1, and A0A8B1JPK4.

It is easy for those skilled in the art to understand that the 3CL protease may further comprise an additional polypeptide to facilitate the expression, purification, and/or tracing of the protein.

In certain embodiments, the additional polypeptide is optionally linked to the N-terminus or C-terminus of the 3CL protease via a linker (e.g., a peptide linker or a small organic molecule linker). Such linkers (e.g., peptide linkers) are well known in the art, examples of which include, but are not limited to, peptide linkers comprising one or more (e.g., 1, 2, 3, 4, or 5) amino acids (e.g., Gly or Ser). In certain embodiments, the peptide linker is flexible. In certain embodiments, a flexible peptide linker may be advantageous, as it is capable of linking two protein/polypeptide components while maintaining their respective activities and functions. Such peptide linkers include, but are not limited to, $(GGGGS)_n$.

In certain embodiments, the additional polypeptide is selected from the group consisting of a tag, a signal peptide or a leader peptide, a detectable label (e.g., Luciferase (fluc), and Green Fluorescent Protein (GFP)), a polypeptide sequence encoding an antibody, a polypeptide sequence encoding a targeting or bioactive protein (e.g., PD1, PD-L1, EGFR, etc.), and any combination thereof.

In certain embodiments, the 3CL protease has an amino acid sequence selected from the group consisting of:

i) a sequence as set forth in SEQ ID NO: 6, 10 or 13;

ii) a sequence with substitution, deletion or addition of one or several amino acids (e.g., substitution, deletion or addition of one, two, three, four or five amino acids) as compared with the sequence as set forth in SEQ ID NO: 6, 10 or 13; and

iii) a sequence with a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 6, 10 or 13.

In certain embodiments, the substitution as described in (ii) is a conservative substitution.

The sequence as set forth in SEQ ID NO: 6, 10 or 13 comprises an amino acid (e.g., methionine (Met)) encoded by an initiation codon (e.g., AUG/ATG) at its N-terminus. Those skilled in the art understand that, in the process of preparing a protein by genetic engineering, due to the function of the initiation codon, the first amino acid of the resulting polypeptide chain is often the amino acid (e.g., Met) encoded by the initiation codon. The 3CL protease of the present application not only comprises amino acid sequences comprising an amino acid (e.g., Met) encoded by an initiation codon at its N-terminus, but also comprises amino acid sequences not comprising an amino acid (e.g., Met) encoded by an initiation codon at its N-terminus. Therefore, the sequence not comprising an amino acid (e.g., Met) encoded by an initiation codon at its N-terminus in the aforementioned amino acid sequences is also within the scope of the present application.

In certain embodiments, the nucleic acid molecule is a DNA molecule or an RNA molecule.

In certain embodiments, the nucleic acid molecule is single-stranded or double-stranded.

In certain embodiments, the nucleic acid molecule is an RNA molecule, preferably an mRNA molecule.

In certain embodiments, the nucleic acid molecule comprises a sequence encoding the 3CL protease.

In certain embodiments, the nucleic acid molecule further comprises one or more selected from the group consisting of a 5'UTR, a Kozak sequence, an initiation codon, a termination codon, a 3'UTR, and a poly-A tail.

In certain embodiments, the nucleic acid molecule comprises, in an order from the 5' end to the 3' end, a 5'UTR, a Kozak sequence, an initiation codon, a sequence encoding the 3CL protease, a termination codon, a 3'UTR, and a poly-A tail.

In certain embodiments, the nucleic acid molecule consists of, in an order from the 5' end to the 3' end, a 5'UTR, a Kozak sequence, an initiation codon, a sequence encoding the 3CL protease, a termination codon, a 3'UTR, and a poly-A tail.

According to the codon degeneracy in the art, in certain embodiments, the sequence encoding the 3CL protease may be replaced according to the codon degeneracy. In certain embodiments, the sequence encoding the 3CL protease is codon-optimized according to the codon bias of a host cell (e.g., a human cell) or non-optimized.

In certain embodiments, the sequence encoding the 3CL protease has a sequence with a sequence identity of at least 30%, at least 35%, at least 40%, at least 45%, at least 50 %, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 1, 8 or 11. In certain embodiments, the sequence encoding the 3CL protease has a sequence as set forth in SEQ ID NO: 1, 8 or 11.

In certain embodiments, the nucleic acid molecule carries a 5' modification (e.g., Cap 0, Cap 1, Cap 2) at its 5' end. In certain embodiments, the nucleic acid molecule carries a 5'Cap 1 type modification at its 5' end.

In certain embodiments, the nucleic acid molecule comprises one or more N1-methylpseuduridine (m1$\psi$) modifications. In certain embodiments, some or all the uracil nucleotide in the nucleic acid molecule is substituted with N1-methylpseudouracil nucleotide.

In certain embodiments, the sequence encoding the 3CL protease carries one or more termination codons at its 3' end.

In certain embodiments, the 5'UTR is a 5'UTR derived from mRNA 1273 from Moderna.

In certain embodiments, the 3'UTR is a 3'UTR derived from mRNA 1273 from Moderna.

In certain embodiments, the 5'UTR has a sequence with a sequence identity of at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 2. In certain embodiments, the 5'UTR has a sequence as set forth in SEQ ID NO: 2.

In certain embodiments, the 3'UTR has a sequence with a sequence identity of at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 4. In certain embodiments, the 3'UTR has a sequence as set forth in SEQ ID NO: 4.

In certain embodiments, the Kozak sequence has a sequence as set forth in SEQ ID NO: 3.

In certain embodiments, the poly-A tail comprises one or more polyadenylic acid sequences, the one or more polyadenylic acid sequences each, independently, consisting of 20 to 120 consecutive adenylic acids; preferably, the poly-A tail comprises multiple polyadenylic acid sequences, and the adjacent polyadenylic acid sequences are linked by a spacer sequence comprising non-A. In certain embodiments, the poly-A tail has a sequence as set forth in SEQ ID NO: 5.

In certain embodiments, the nucleic acid molecule has a sequence with a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 7, 9 or 12. In certain embodiments, the nucleic acid molecule has a sequence as set forth in SEQ ID NO: 7, 9 or 12.

A second aspect of the present application provides an isolated nucleic acid molecule, comprising a nucleotide sequence encoding a 3CL protease, wherein the 3CL protease is as defined in the first aspect of the present application.

In certain embodiments, the isolated nucleic acid molecule is the nucleic acid molecule as defined in the first aspect of the present application. In certain embodiments, the isolated nucleic acid molecule encodes the 3CL protease as defined in the first aspect of the present application.

A third aspect of the present application provides a vector, comprising the isolated nucleic acid molecule of the second aspect of the present application.

In certain embodiments, the vector of the present application is, for example, a plasmid, a cosmid, bacteriophage, or a lentivirus.

In certain embodiments, the vector is a cloning vector or an expression vector.

In certain embodiments, the vector is capable of expressing the 3CL protease as described in the first aspect in a subject.

In certain embodiments, the vector is a DNA vector or an RNA vector.

A fourth aspect of the present application provides a delivery composition, comprising a delivery vehicle, and one or more selected from the group consisting of: the 3CL protease of the first aspect of the present application, the isolated nucleic acid molecule of the second aspect of the present application, and the vector of the third aspect of the present application.

In certain embodiments, the delivery vehicle is used for encapsulating, carrying, or delivering the 3CL protease, the isolated nucleic acid molecule, or the vector.

In certain embodiments, the delivery vehicle comprises a non-viral vector, a viral vector, and a virus-like particle (VLP) vector between viral vector and non-viral vector.

In certain embodiments, the non-viral vector includes, but is not limited to, cationic liposome, lipid nanoparticle, lipid polymer, artificial microsphere, micelle, lipid-polymer hybrid system, extracellular vesicle, natural or engineered exosome, and the like. In certain embodiments, the viral vector includes, but is not limited to, adeno-associated virus, lentivirus, adenovirus, retrovirus, vaccinia virus, measles virus, herpes simplex virus, alphavirus, vesicular stomatitis virus, influenza virus, and the like. In certain embodiments, the VLP vector includes, but is not limited to, VLP-mRNA delivery vehicle that utilizes the principle of specific recognition between an mRNA stem-loop structure and a bacteriophage capsid protein.

In certain embodiments, the delivery vehicle is a lipid nanoparticle.

A fifth aspect of the present application provides a pharmaceutical composition, comprising the 3CL protease of the first aspect of the present application, the isolated nucleic acid molecule of the second aspect of the present application, the vector of the third aspect of the present application and/or the delivery composition of the fourth aspect of the present application, and a pharmaceutically acceptable carrier and/or excipient.

In certain embodiments, the pharmaceutical composition further comprises an additional anti-tumor agent.

In certain embodiments, the additional anti-tumor agent is selected from the group consisting of small-molecule chemical drug, biomacromolecule antibody or protein, chimeric antigen receptor cell-based drug, and antibody-drug conjugate.

In certain embodiments, the pharmaceutical composition is administered orally, by spray inhalation, rectally, nasally, buccally, vaginally, topically, parenterally such as by subcutaneous, intravenous, intramuscular, intraperitoneal, intrathoracic, intrathecal, intraventricular, intrasternal and intracranial injection or infusion, intratracheal installation, surgical implantation, transdermal delivery, local injection, bolus injection/bombardment, or via an explanted reservoir. In certain embodiments, the pharmaceutical composition is administered orally, intraperitoneally, intrathoracically, or intravenously.

A sixth aspect of the present application provides a method of inhibiting a tumor cell *in vivo* or *in vitro,* comprising: contacting the 3CL protease of the first aspect of the present application, the isolated nucleic acid molecule of the second aspect of the present application, the vector of the third aspect of the present application, the delivery composition of the fourth aspect of the present application or the pharmaceutical composition of the fifth aspect of the present application with the tumor cell or delivering them to the tumor cell.

A seventh aspect of the present application provides a use of the isolated nucleic acid molecule of the second aspect of the present application, the vector of the third aspect of the present application, the delivery composition of the fourth aspect of the present application or a pharmaceutical composition of the fifth aspect of the present application in the preparation of a medicament for the prevention and/or treatment of a tumor.

An eighth aspect of the present application provides a use of the 3CL protease of the first aspect of the present application, the isolated nucleic acid molecule of the second aspect of the present application, the vector of the third aspect of the present application, the delivery composition of the fourth aspect of the present application or the pharmaceutical composition of the fifth aspect of the present application in the prevention and/or treatment of a tumor.

A ninth aspect of the present application provides a method of the prevention and/or treatment of a tumor in a subject, comprising administrating the 3CL protease of the first aspect of the present application, the isolated nucleic acid molecule of the second aspect of the present application, the vector of the third aspect of the present application, the delivery composition of the fourth aspect of the present application or the pharmaceutical composition of the fifth aspect of the present application to the subject in need thereof. In certain embodiments, the method further comprises administering a second therapy to the subject, wherein the second therapy is selected from the group consisting of surgery, chemotherapy, radiotherapy, immunotherapy, gene therapy, DNA therapy, RNA therapy, nanotherapy, virotherapy, adjuvant therapy, and any combination thereof; optionally, the second therapy may be applied simultaneously, separately or sequentially.

A tenth aspect of the present application provides a medicament or composition for the prevention and/or treatment of a tumor in a subject, comprising the 3CL protease of the first aspect of the present application, the isolated nucleic acid molecule of the second aspect of the present application, the vector of the third aspect of the present application, the delivery composition of the fourth aspect of the present application or the pharmaceutical composition of the fifth aspect of the present application.

An eleventh aspect of the present application provides the 3CL protease of the first aspect of the present application, the isolated nucleic acid molecule of the second aspect of the present application, the vector of the third aspect of the present application, the delivery composition of the fourth aspect of the present application or the pharmaceutical composition of the fifth aspect of the present application for use in the prevention and/or treatment of a tumor in a subject.

In certain embodiments, the tumor is a solid tumor or a hematological tumor (such as leukemia, lymphoma, and myeloma).

In certain embodiments, the tumor is a solid tumor.

In certain embodiments, the tumor is selected from the group consisting of pancreatic cancer, gastric cancer, liver cancer, melanoma, colorectal cancer, renal cancer, cervical cancer, osteosarcoma, prostatic cancer, glioma, and lung cancer.

In certain embodiments, the tumor cell is a cell of a solid tumor or a hematological tumor (such as leukemia, lymphoma, and myeloma).

In certain embodiments, the tumor cell is selected from the group consisting of pancreatic cancer cell, gastric cancer cell, liver cancer cell, melanoma cell, colorectal cancer cell, renal cancer cell, cervical cancer cell, osteosarcoma cell, prostatic cancer cell, glioma cell, and lung cancer cell.

In certain embodiments, types of the pancreatic cancer include, but are not limited to, pancreatic acinar cell carcinoma, adenosquamous carcinoma, squamous cell carcinoma, signet-ring cell carcinoma, undifferentiated carcinoma, undifferentiated carcinoma with giant cells, ampullary carcinoma, pancreatic neuroendocrine tumor, and the like. In certain embodiments, types of the gastric cancer include, but are not limited to, cardiac cancer, gastric body cancer and pylorus cancer. In certain embodiments, types of the liver cancer include, but are not limited to, hepatocellular carcinoma, hepatocellular liver cancer, fibrolamellar liver cancer, intrahepatic cholangiocarcinoma, angiosarcoma, hepatoblastoma, secondary liver cancer, and benign liver tumor. In certain embodiments, types of the melanoma include, but are not limited to, localized melanoma, regional melanoma, and distant metastatic melanoma. In certain embodiments, types of the colorectal cancer include, but are not limited to, colon cancer and rectal cancer, specifically including adenocarcinoma, undifferentiated carcinoma, adenosquamous carcinoma, squamous cell carcinoma, small cell carcinoma, carcinoid, mucinous carcinoma, and the like. In certain embodiments, types of the renal cancer include, but are not limited to, clear cell carcinoma, papillary cell carcinoma, chromophobe cell carcinoma, multilocular cystic renal cell neoplasm of low malignant potential, collecting duct carcinoma, and renal medullary carcinoma. In certain embodiments, types of the cervical cancer include, but are not limited to, squamous carcinoma, adenocarcinoma, and adenosquamous carcinoma. In certain embodiments, types of the osteosarcoma include, but are not limited to, telangiectatic osteosarcoma, small round cell osteosarcoma, fibrohistiocytic osteosarcoma, intramedullary well-differentiated osteosarcoma, multicentric osteosarcoma, intracortical osteosarcoma, parosteal osteosarcoma, dedifferentiated parosteal osteosarcoma, periosteal osteosarcoma, and high-grade surface osteosarcoma. In certain embodiments, types of the prostatic cancer include, but are not limited to, adenocarcinoma (acinar adenocarcinoma), ductal adenocarcinoma, urothelial carcinoma, squamous cell carcinoma, and adenosquamous carcinoma. In certain embodiments, types of the glioma include, but are not limited to, PAs, astrocytoma, oligoastrocytoma, anaplastic astrocytoma, anaplastic oligoastrocytoma, and glioblastoma. In certain embodiments, types of the lung cancer include, but are not limited to, small cell lung cancer and non-small cell lung cancer (adenocarcinoma, squamous cell carcinoma, large cell carcinoma, adenosquamous carcinoma, carcinoid, and the like).

Further, according to the progression status of each type of tumor, the tumor includes, but are not limited to, early-stage, mid-stage, late-stage tumor or stage I, II, III, or IV tumor.

The medicament, composition or pharmaceutical composition of the present application may be formulated into any dosage form known in the medical field, such as tablet, pill, suspension, emulsion, solution, gel, capsule, powder, granule, elixir, lozenge, suppository, injection (including solution for injection, sterile powder for injection and concentrated solution for injection), inhalant, spray, and the like. Preferred dosage form depends on the intended administration route and therapeutic purpose. The medicament, composition or pharmaceutical composition of the application should be sterile and stable under the conditions of manufacture and storage. One preferred dosage form is an injection. Such an injection may be a sterile injection solution. For example, the sterile injection solution may be prepared by the following method: incorporating a required dose of the pharmaceutical composition of the present application into an appropriate solvent, and optionally, simultaneously incorporating other desired ingredients (including but not limited to, pH regulator, surfactant, adjuvant, ionic strength enhancer, isotonic agent, preservative, diluent, or any combination thereof), followed by filtration and sterilization. In addition, the sterile injectable solution may be prepared as a sterile lyophilized powder (e.g., by vacuum drying or freeze-drying) for easy storage and use. Such a sterile lyophilized powder can be dispersed in a suitable carrier, such as sterile pyrogen-free water, prior to use.

Furthermore, the 3CL protease, nucleic acid molecule encoding the 3CL protease, vector or delivery composition of the present application may be present in the medicament, the composition or the pharmaceutical composition in the form of a unit dose for easy administration.

The medicament, composition or pharmaceutical composition of the present application may be administered by any suitable routes known in the art, including, but being not limited to, oral administration, spray inhalation, rectal administration, nasal administration, buccal administration, vaginal administration, topical administration, parenteral administration such as subcutaneous, intravenous, intramuscular, intraperitoneal, intrathoracic, intrathecal, intraventricular, intrasternal and intracranial injection or infusion, intratracheal installation, surgical implantation, transdermal delivery, local injection, and bolus injection/bombardment, or administration via an explanted reservoir. For many therapeutic uses, preferred administrate route/way is oral, intraperitoneal or intravenous. Those skilled in the art should understand that the administration route and/or way will vary depending on intended purpose.

The medicament, composition or pharmaceutical composition of the present application may comprise a "therapeutically effect amount" or a "prophylactically effective amount" of the 3CL protease, nucleic acid molecule encoding the 3CL protease, vector or delivery composition of the present application. The "prophylactically effective amount" refers to an amount sufficient to prevent, inhibit, or delay the occurrence of a disease. The "therapeutically effect amount" refers to an amount sufficient to cure or at least partially inhibit a disease and complications thereof in a patient with this disease. The therapeutically effective amount of the 3CL protease, nucleic acid molecule encoding the 3CL protease, vector, or delivery composition of the present application may vary depending on the following factors: the severity of a disease to be treated, the general state of the patient's own immune system, the patient's general conditions such as age, weight and gender, the administration route of drug, and additional concurrently-administered treatments.

In the present application, the dosage regimen may be adjusted to achieve the optimal intended response (e.g., a therapeutic or prophylactic response). For example, a single administration can be given, multiple administrations may be given over a period of time, or the dose may be reduced or increased proportionally depending on the urgency of the treatment situation.

In certain embodiments, the isolated nucleic acid molecule, such as an RNA molecule, may be prepared by *in vitro* transcription, or may be prepared by using one or more combinations of an oligonucleotide synthesizer, host cell expression or any other method known in the art.

Specifically, an *in vitro* transcription template plasmid can be first constructed, and *in vitro* transcription can be performed by simulating the intracellular transcription environment.

The basic structure of the *in vitro* transcription template plasmid comprises multiple polynucleotide functional regions. The 5' end of the plasmid template may comprise a promoter sequence responsible for the initiation of transcription *in vitro* or *in vivo.* The 5' end of the plasmid template may comprise one or more complete or incomplete 5' untranslated regions (UTRs), such as natural 5'UTRs and non-natural 5'UTRs encoding mRNA, including, but not being limited to, heterologous 5'UTRs or synthetic 5'UTRs. The 5' end of the plasmid template may comprise one or more signal sequence regions. The 3' end of the plasmid template may comprise one or more complete or incomplete 3'UTRs, such as natural 3'UTRs and non-natural 3'UTRs encoding mRNA, including, but not being limited to, heterologous 3'UTRs or synthetic 3'UTRs. An Open Reading Frame (ORF) that can encode at least one polypeptide or protein of interest should be comprised between 5'UTR and 3'UTR of the plasmid template. The 3' end of the plasmid template may comprise a 3' tailing sequence. The 3' tailing sequence may be, but is not limited to, a poly-A tail, a poly-A-G-quadruplex and/or a stem-loop sequence. The regulatory features of the UTR can be incorporated into the polynucleotides of the present application to enhance the stability of the molecule.

The present application provides 5'UTR and 3'UTR nucleotide sequences used in some examples, which do not limit the scope of protection of the present application. Those skilled in the art will understand that, in certain embodiments, commonly used variants of 5'UTR, 3'UTR sequence may be added to an *in vitro* transcription template plasmid, followed by the transcription of mRNA including a corresponding codon. One or more nucleotides in the 5'UTR, 3'UTR sequence variants may be added to or removed from the terminus. Furthermore, the 5'UTR, 3'UTR sequence variants can be altered in the same orientation or position as that of transcripts from which they are selected, and thus, the 5'UTR, 3'UTR sequences may be inverted, extended, shorten, or prepared together with one or more other 5'UTR, 3'UTR sequences. Methods that can achieve the purpose of the present application of performing in vitro transcription and translation of the optimized mRNA molecules using 5'UTR, 3'UTR sequences with different nucleotide combinations are all within the scope of protection of this application.

In other aspects, the 5'UTR and the 3'UTR can be derived from different species. The UTR may further comprise a translational enhancer element (TEE).

The *in vitro* transcription template plasmid comprising an isolated nucleic acid molecule (e.g., a sequence encoding a 3CL protease of coronavirus (including SARS-CoV, MERS-CoV, SARS-CoV-2 and the like)) according to the present application may comprise a promoter element, such as a T7 promoter. In certain embodiment, the T7 promoter sequence is preferably 5'-TAATACGACTCACTATA-3'. As a non-limiting example, other promoter sequences that can replace the T7 promoter sequence and enable efficient transcription of functional RNA under the guidance of RNA polymerase, such as a Sp6 promoter, and a Syn5 promoter, or those can be used for the synthesis of the RNA of the present application, are within the protection scope of the present application.

The *in vitro* transcription template plasmid comprising an isolated nucleic acid molecule (e.g., a sequence encoding a 3CL protease of coronavirus (including SARS-CoV, MERS-CoV, SARS-CoV-2 and the like)) and the isolated nucleic acid molecule according to the present application may further comprise an element similar to or functioning similarly to an initiation codon region. In certain embodiments, the nucleotide sequence of the initiation codon is preferably ATG, corresponding to the sequence of the initiation codon in the RNA, AUG, which can achieve high translation efficiency. As a non-limiting example, the translation of the polynucleotide may be initiated at a codon other than the initiation codon AUG, i.e., an alternative initiation codon. Thus, the nucleotide sequence of the initiation codon can also be, but is not limited to, ACG, AGG, AAG, CTG/CUG, GTG/GUG, ATA/AUA, ATT/AUU, and TTG/UUG.

The *in vitro* transcription template plasmid comprising an isolated nucleic acid molecule (e.g., a sequence encoding a 3CL protease of coronavirus (including SARS-CoV, MERS-CoV, SARS-CoV-2 and the like)) and the isolated nucleic acid molecule according to the present application may further comprise at least one or two or more consecutive termination codon elements, located upstream of the 3'UTR, and the nucleotide sequence of the termination codon may selected from the group consisting of UGA, UAA, UAG and any combination thereof. In certain embodiments, the nucleotide sequence of the termination codon is preferably a single UGA. In certain embodiments, the nucleotide sequence of the termination codon is preferably UGAUAAUAG.

The isolated nucleic acid molecule (e.g., a sequence encoding a 3CL protease of coronavirus (including SARS-CoV, MERS-CoV, SARS-CoV-2 and the like)) according to the present application may further comprise a polyadenylation tailing sequence, located immediately downstream of the 3'UTR element, to enhance the stability of the nucleic acid molecule. The polyadenylation tail may be present or absent in a circular *in vitro* transcription template plasmid, and may be transcribed together with the template or added after the transcription. The length of the tailing sequence is designed depending on the length of the overall nucleic acid molecule or the length of a specific region. Such design may be based on the length of the coding region, the length of a specific feature or region, or based on the length of the final product which is expressed by polynucleotide. It should also be understood that the 3' tailing sequence can be, but is not limited to, a poly-A tail, a poly-A-G-quadruplex, and/or a stem-loop sequence, and the design of the tailing sequence can be artificially optimized to achieve the goal of maximally protecting a nucleic acid molecule (e.g., an RNA molecule) from degradation. In certain embodiments, the preferred length of the tailing sequence is 110 nt. As a non-limiting example, the tailing sequence according to the present application does not limit the protection scope of the present application.

The *in vitro* transcription template plasmid comprising an isolated nucleic acid molecule (e.g., a sequence encoding a 3CL protease of coronavirus (including SARS-CoV, MERS-CoV, SARS-CoV-2 and the like)) and the isolated nucleic acid molecule according to the present application may further comprise a tag element. Generally, the nucleotide sequence of the tag element is located immediately downstream of the coding sequence (CDS) of the gene, and correspondingly in the nucleic acid molecule, the codon sequence of the tag element is immediately downstream of the codon of the coding sequence. Once the codon of the encoding sequence is translated, the codon of the tag element is also translated. The tag element may be a fluorescent protein nucleotide sequence including, but not being limited to, nucleotide sequences of green fluorescent protein, red fluorescent protein, and firefly luciferase. Transcription and translation of the tag sequence facilitate monitoring the expression of the nucleic acid molecule of interest in cells and *in vivo.*

The *in vitro* transcriptional template plasmid comprising an isolated nucleic acid molecule (e.g., a sequence encoding a 3CL protease of coronavirus (including SARS-CoV, MERS-CoV, SARS-CoV-2 and the like)) and the isolated nucleic acid molecule according to the present application may further comprise a signal peptide element. Generally, the nucleotide sequence of the signal peptide element is located between the 5'UTR and CDS region, and correspondingly in the RNA, the codon sequence of the signal peptide element is located between 5'UTR codon and CDS codon. The nucleotide sequence of the signal peptide element may be encoded, and its transcription and translation facilitate the provision of intracellular and extracellular localization signals for the polypeptide sequence translated from the RNA.

The isolated nucleic acid molecule (e.g., a sequence encoding a 3CL protease of coronavirus (including SARS-CoV, MERS-CoV, SARS-CoV-2 and the like)) according to the present application may further comprise a 5'cap structure capable of binding to an RNA cap binding protein (CBP), to enhance the stability of the RNA. The capping way may be a way of capping after the transcription using a capping enzyme, or a way of co-transcription capping by adding a cap analogue into an in vitro transcription reaction system. The cap analogue may be chemically (i.e. non-enzymatically) or enzymatically synthesized and/or linked to the nucleotide sequence of the present application, such as an anti-reverse cap analogue (ARCA), a mCap or a Cap1. As a non-limiting example, the present application preferably employs the co-transcription capping method, to add a Cap1 to the RNA. Different cap structures may be also added to the isolated nucleic acid molecule (e.g., a sequence encoding a 3CL protease of coronavirus (including SARS-CoV, MERS-CoV, SARS-CoV-2 and the like)) in other ways, to achieve effective translation of the isolated nucleic acid molecule.

The isolated nucleic acid molecule (e.g., a sequence encoding a 3CL protease of coronavirus (including SARS-CoV, MERS-CoV, SARS-CoV-2 and the like)) according to the present application may further comprise a nucleotide sequence encoding one or more other heterologous polypeptides for improving the pharmacokinetic or pharmacodynamic properties, such as extending half-life, of the isolated nucleic acid molecule and a translation product thereof.

In certain embodiments, the isolated nucleic acid molecule may be synthesized by using a specific chemically modified nucleotide, which can reduce the interaction of the molecule of interest with an innate immune receptor, lower its inherent immunogenicity, and optimize the the translation efficiency of the nucleic acid molecule into protein.

In certain embodiments, a modified nucleotide, N1-methylpseuduridine (N1-me-pseudo U, m1Ψ), is used to replace a natural uridine U as one of the transcription materials, but this does not mean that the mRNA sequence protected by the present application is limited to this nucleotide modification. When the isolated nucleic acid molecule of the present application is prepared by the *in vitro* transcription using the *in vitro* transcription template plasmid according to the present application or by using an oligonucleotide synthesizer, by using host cell expression system or by using any one or a combination of two or more other method known in the art, artificially optimized isolated nucleic acid molecules of the present application can be obtained by modifying and replacing the four natural nucleotide substrates A, C, G, and U. For example, specific nucleotide replacement methods may be, but is not limited to, replacing natural adenosine with N1-methyladenosine (m1A) or N6-methyladenosine (m6A), replacing natural cytidine with 5-methylcytidine (m5C), and replacing natural uridine with 5-methyluridine (m5U), s2U, 5-methoxyuridine (5moU), pseudouridine (ψ), N1-methylpseudouridine (m1Ψ), etc.

In general, a sequence optimized nucleic is produced by at least one step that includes replacing a codon in a reference sequence with a synonymous codon (i.e., a codon encoding the same amino acid).

Those skilled in the art will understand that the T base in the codon chart of the present application are present in DNA, and the T base will be replaced by the corresponding U base in RNA. For example, the T base of a codon-nucleotide sequence in the form of DNA (for example, a vector or an *in vitro* translation (IVT) template) of the present application will be transcribed into a U base in its corresponding transcription mRNA. In this regard, both a codon-optimized DNA sequence (comprising T) and its corresponding RNA sequence (comprising U) are considered to be the codon-optimized nucleotide sequence of the present application. For example, the DNA and/or RNA reference sequences provided by the present application can be optimized by replacing all codons encoding a certain amino acid with only one of the alternative codons provided in the codon chart. Those skilled in the art will also understand that an equivalent codon chart can be generated by replacing one or more bases with non-natural bases. Thus, for example, a TTC codon (DNA chart) would correspond to a UUC codon (RNA chart), which further will correspond to a ΨΨC codon (RNA chart in which U has been replaced by a pseudouridine).

Further, the sequence-optimized nucleic acid molecules and *in vitro* transcription plasmid template of the present application may be generated by one or more codon optimization methods or a combination thereof.

The isolated nucleic acid molecule (e.g., a sequence encoding a 3CL protease of coronavirus (including SARS-CoV, MERS-CoV, SARS-CoV-2 and the like)) according to the present application may may be circular or in a circular shape. A circular nucleic acid molecule means that the ends are linked in some way, whether by linkage, covalent bonds, co-association with same protein or other molecule or complex, or by hybridization.

The isolated nucleic acid molecule (e.g., a sequence encoding a 3CL protease of coronavirus (including SARS-CoV, MERS-CoV, SARS-CoV-2 and the like)) according to the present application may be designed to conjugate with the following: other polynucleotides, dyes, intercalating agents (e.g., acridine), crosslinking agents (e.g., psoralene, and mitomycin C), porphyrins (TPPC4, texaphyrin, Sapphyrin, polycyclic aromatic hydrocarbons (e.g., phenazine, and dihydrophenazine), artificial endonucleases (e.g., EDTA), alkylating agents, phosphates, amino, sulfhydryl, PEG (e.g., PEG-40K), MPEG, [MPEG]$_2$, polyamino, alkyl, substituted alkyl, radiolabeled markers, enzymes, haptens (e.g., biotin), transportation/absorption enhancers (e.g., aspirin, vitamin E, and folic acid), synthetic ribonucleases, proteins (e.g., glycoproteins) or peptides (e.g., molecules with specific affinity to co-ligand) or antibodies (molecules with antibodies binding to specified cell types such as cancer cells, endothelial cells, or bone cells), hormones and hormone receptors, non-peptide species such as lipids, lectins, carbohydrates, vitamins, cofactors or drugs. The conjugation may result in increased stability and/or half-life and it is particularly suitable for enabling polynucleotides to target cells, tissues or specific sites in organisms.

**Term Definitions**

In the present application, unless otherwise stated, the scientific and technical terms used herein have the meanings commonly understood by those skilled in the art. In addition, operation steps for virology, cell culture, biochemistry, cell biology, nucleic acid chemistry, and the like used herein are conventional steps widely used in corresponding fields. Meanwhile, in order to better understand the present application, definitions and explanations of relevant related terms are provided below.

When the terms "for example," "e.g.," "such as," "comprising," "including" or their variants are used herein, these terms shall not be regarded as restrictive terms, but shall be interpreted as meaning "but not limited to" or "not limited to."

Unless otherwise indicated herein or clearly contradicted by context, the terms "a" and "an" and "the" and similar referents in the context of describing the present application (especially in the context of the following claims) shall be construed to cover both the singular and the plural.

As used herein, the term "and/or" shall be construed as a specific disclosure of each of two or more specified features or elements, as well as any combination of two or more features or elements. Thus, the term "and/or" used in phrases such as "A and/or B" is intended to include "A and B", "A or B", "A" (alone), and "B" (alone).

As used herein, the term "coronavirus" is a single-stranded positive-sense RNA virus with spikes protruding from its envelope, forming a crown-like appearance. The coronavirus includes, but is not limited to, MERS-CoV, SARS-CoV-2, SARS-CoV, HCoV-HKU1, HCoV-OC43, HCoV-229E, HCoV-NL63, or their variants. The SARS-CoV-2 comprises both the original strain of SARS-CoV-2 and variants thereof, such as Alpha, Beta, Gamma, Delta and Omicron variants.

As used herein, the term "identity" refers to the degree of sequence match between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The "percent identity" between two sequences is a function of the number of identical sites shared by the two sequences over the total number of sites for comparison × 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison is performed when the two sequences are aligned to yield maximum identity. Such alignment can be achieved using, for example, the method of Needleman et al. (J. Mol. Biol. 48:443-453, 1970), which can be conveniently carried out by computer programs such as the Align program (DNAstar, Inc.). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, and with a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and with a gap weight of 16, 14, 12, 10, 8, 6, or 4 and a length weight of 1, 2, 3, 4, 5, or 6.

The writing of the twenty conventional amino acids involved herein follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present application, the terms "polypeptide" and "protein" have the same meaning, and are used interchangeably. Furthermore, in the present application, amino acids are generally represented by single-letters and three-letter abbreviations well known in the art. For example, alanine can be represented by A or Ala.

As used herein, the term "conservative substitution" refers to amino acid substitutions which would not disadvantageously affect or change the essential properties of a protein/polypeptide comprising the amino acid sequence. For example, a conservative substitution may be introduced by standard techniques known in the art such as site-directed mutagenesis and PCR-mediated mutagenesis. Conservative amino acid substitutions include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having basic side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, and tryptophan), amino acids having nonpolar side chains (for example, alanine, valine, leucine, isoleucine, proline, phenylalanine, and methionine), amino acids having β-branched side chains (such as threonine, valine, and isoleucine) and amino acids having aromatic side chains (for example, tyrosine, phenylalanine, tryptophan, and histidine). Therefore, generally a conservative substitution refers to a substitution of a corresponding amino acid residue with another amino acid residue from the same side-chain family. Methods for identifying amino acid conservative substitutions are well known in the art (see, for example, Brummell et al., Biochem. 32: 1180-1187 (1993); Kobayashi et al., Protein Eng. 12(10): 879-884 (1999); and Burks et al., Proc. Natl. Acad. Sci. USA 94: 412-417 (1997), which are incorporated herein by reference).

As used herein, the term "pharmaceutically acceptable carrier and/or excipient" refers to a carrier and/or excipient that is pharmacologically and/or physiologically compatible with subjects and active ingredients, which is well known in the art (see, for example, Remington's Pharmaceutical Sciences, Edited by Gennaro AR, 19th ed. Pennsylvania:

Mack Publishing Company, 1995), and includes, but is not limited to, pH regulators, surfactants, ionic strength enhancers, agents for maintaining osmotic pressure, agents for delaying absorption, diluents, adjuvants, preservatives, stabilizers and the like. For example, the pH regulators include, but are not limited to, phosphate buffers. The surfactants include, but are not limited to, cationic surfactants, anionic surfactants or nonionic surfactants, such as Tween-80. The ionic strength enhancers include but are not limited to sodium chloride. The agents for maintaining osmotic pressure include, but are not limited to, sugars, NaCl, and analogues thereof. The agents for delaying absorption include, but are not limited to, monostearate and gelatin. The diluents include, but are not limited to, water, aqueous buffer (e.g., buffered saline), alcohol and polyol (e.g., glycerol), and the like. The adjuvants include, but are not limited to, aluminum adjuvants (e.g., aluminum hydroxide), Freund's adjuvants (e.g., complete Freund's adjuvants), and the like. The preservatives include, but are not limited to, various antibacterial agents and antifungal agents, such as thiomersal, 2-phenoxyethanol, parabens, chlorobutanol, phenol, sorbic acid, and the like. The stabilizers have the meanings as commonly understood by those skilled in the art, which is capable of stabilizing the desired activities (e.g., oncolytic activity) of active ingredients in a drug, and include, but are not limited to, sodium glutamate, gelatin, SPGA, sugars (e.g., sorbitol, mannitol, starch, sucrose, lactose, dextran, or glucose), amino acids (e.g., glutamic acid, or glycine), proteins (e.g., dried whey, albumin, or casein) or degradation products thereof (e.g., lactalbumin hydrolysate), and the like.

As used herein, the term "prevention" refers to a method implemented to prevent or delay the occurrence of a disease or condition or symptom (e.g., a tumor) in a subject. As used herein, the term "treatment" refers to a method implemented to obtain a beneficial or desired clinical result. For the purposes of the present application, beneficial or desired clinical results include, but are not limited to, alleviation of symptoms, diminishment of the extent of the disease, stabilization (i.e., no longer worsening) of the state of the disease, delay or slowing of the progression of the disease, improvement or alleviation of the state of the disease, and relief of symptoms (whether partial or complete), whether detectable or undetectable. In addition, "treatment" may also refer to prolongation of survival as compared to the expected survival if not receiving treatment.

As used herein, the term "effective amount" refers to an amount which can effectively achieve intended purposes. For example, a therapeutically effective amount may be an amount effective or sufficient to treat or cure a disease (for example, a tumor), delay the occurrence of symptoms of a disease (for example, a tumor), and/or delay the progression of a disease (for example, a tumor). Such an effective amount can be readily determined by those skilled in the art or a physician, and it can be related to intended purposes (for example, treatment), the subject's general health status, age, gender, weight, severity of the disease to be treated, complications, route of administration, and the like. Determination of such effective amounts is well within the capability of those skilled in the art.

As used herein, the term "subject" refers to a mammal, for example, a primate mammal, such as human. In certain embodiments, the subject (for example, a human) has a tumor or is at risk of developing a tumor.

As used herein, the terms "cancer" and "tumor" may be used interchangeably, and refer to a broad class of diseases characterized by uncontrolled growth of abnormal cells in the body. Uncontrolled cell division may lead to the formation of malignant tumors or cells that invade adjacent tissues and may metastasize to distant parts in the body through the lymphatic system or blood stream. Cancers include benign and malignant cancers as well as dormant tumors or micrometastases. Cancers also include hematological malignancies, such as lymphoma, leukemia, myeloma or lymphoid malignancies, as well as spleen cancer and lymph node tumors.

## Beneficial Effects of the present Application

The 3CL protease of coronavirus (such as SARS-CoV-2) or the nucleic acid molecule encoding the 3CL protease provided in the present application has a broad-spectrum, significant tumor cell killing capacity, and can be used for the prevention and/or treatment of a tumor.

## Brief Description of the Drawings

The embodiments of the present application will be described in detail below with reference to the drawings and examples. However, those skilled in the art will understand that the following drawings and examples are for illustrative purposes only and not intended to limit the scope of the present application. According to the following detailed description of the drawings and preferred embodiments, the various objects and advantages of the present application will become apparent to those skilled in the art.

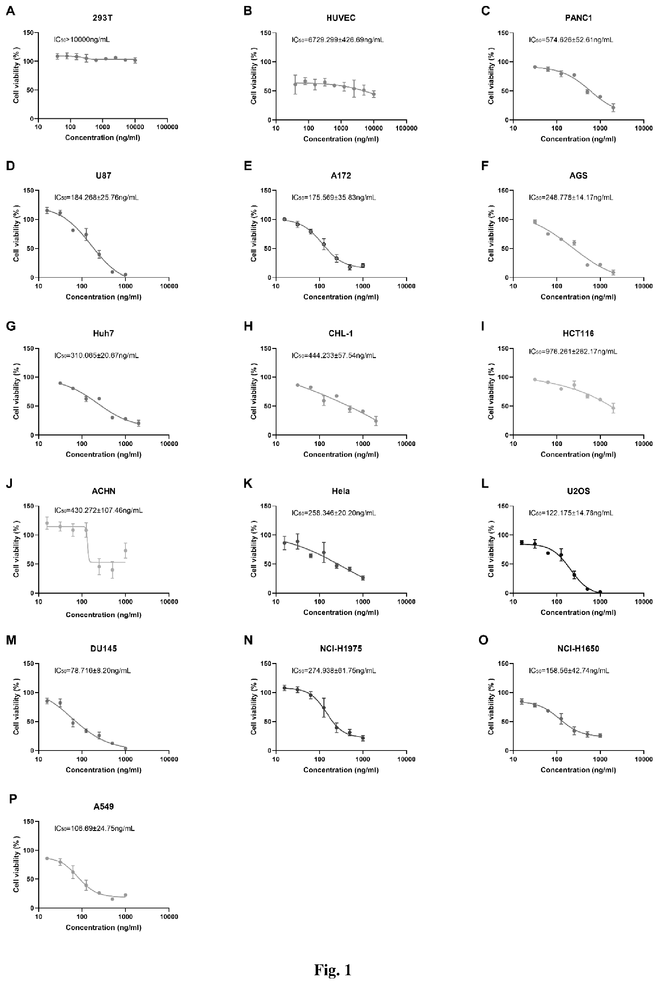

Fig. 1 shows the effects of mRNA encoding 3CL protease of SARS-CoV-2 on the viability of various normal cell lines and tumor cell lines. Specifically, panel A shows the effect of mRNA encoding 3CL protease of SARS-CoV-2 on the viability of the normal human embryonic kidney cell line (HEK 293T); panel B shows the effect of mRNA encoding 3CL protease of SARS-CoV-2 on the viability of the human umbilical vein endothelial cell line HUVEC; panel C shows the effect of mRNA encoding 3CL protease of SARS-CoV-2 on the viability of the human pancreatic cancer cell line PANC-1; panel D shows the effect of mRNA encoding 3CL protease of SARS-CoV-2 on the viability of the human glioblastoma cell line U87;

panel E shows the effect of an mRNA encoding 3CL protease of SARS-CoV-2 on the viability of the human glioblastoma cell line A172; panel F shows the effect of mRNA encoding 3CL protease of SARS-CoV-2 on the viability of human gastric cancer cell line AGS; panel G shows the effect of an mRNA encoding 3CL protease of SARS-CoV-2 on the viability of human hepatocellular carcinoma cell line Huh7; panel H shows the effect of an mRNA encoding 3CL protease of SARS-CoV-2 on the viability of human melanoma cell line CHL-1; panel I shows the effect of an mRNA encoding 3CL protease of SARS-CoV-2 on the viability of human colon cancer cell line HCT 116; panel J shows the effect of an mRNA encoding 3CL protease of SARS-CoV-2 on the viability of human renal cancer cell line ACHN; panel K shows the effect of an mRNA encoding 3CL protease of SARS-CoV-2 on the viability of human cervical cancer cell line Hela; panel L shows the effect of an mRNA encoding 3CL protease of SARS-CoV-2 on the viability of human osteosarcoma cell line U2OS; panel M shows the effect of an mRNA encoding 3CL protease of SARS-CoV-2 on the viability of human prostatic cancer cell line DU145; panel N shows the effect of an mRNA encoding 3CL protease of SARS-CoV-2 on the viability of human lung cancer cell line NCI-H1975; panel O shows the effect of an mRNA encoding 3CL protease of SARS-CoV-2 on the viability of human lung cancer cell line NCI-H1650; panel P shows the effect of an mRNA encoding 3CL protease of SARS-CoV-2 on the viability of human lung cancer cell line A549.

**Sequence Information**

The information of partial sequences involved in the present disclosure is shown in the following table A.

Table A: Information of partial sequences

| SEQ ID NO: | Description of sequences | Sequence information |
|---|---|---|
| 1 | Nucleotide sequences encoding 3CL protease of SARS-CoV-2 | AGUGGUUUUAGAAAAAUGGCAUUCCCAUCUGGUAAAGU UGAGGGUUGUAUGGUACAAGUAACUUGUGGUACAACUA CACUUAACGGUCUUUGGCUUGAUGACGUAGUUUACUGU CCAAGACAUGUGAUCUGCACCUCUGAAGACAUGCUUAA CCCUAAUUAUGAAGAUUUACUCAUUCGUAAGUCUAAUC AUAAUUUCUUGGUACAGGCUGGUAAUGUUCAACUCAGG GUUAUUGGACAUUCUAUGCAAAAUUGUGUACUUAAGCU UAAGGUUGAUACAGCCAAUCCUAAGACACCUAAGUAUA AGUUUGUUCGCAUUCAACCAGGACAGACUUUUUCAGUG UUAGCUUGUUACAAUGGUUCACCAUCUGGUGUUUACCA AUGUGCUAUGAGGCCCAAUUUCACUAUUAAGGGUUCAU UCCUUAAUGGUUCAUGUGGUAGUGUUGGUUUUAACAUA GAUUAUGACUGUGUCUCUUUUUGUUACAUGCACCAUAU GGAAUUACCAACUGGAGUUCAUGCUGGCACAGACUUAG AAGGUAACUUUUAUGGACCUUUUGUUGACAGGCAAACA GCACAAGCAGCUGGUACGGACACAACUAUUACAGUUAA UGUUUUAGCUUGGUUGUACGCUGCUGUUAUAAAUGGAG ACAGGUGGUUUCUCAAUCGAUUUACCACAACUCUUAAU GACUUUAACCUUGUGGCUAUGAAGUACAAUUAUGAACC UCUAACACAAGACCAUGUUGACAUACUAGGACCUCUUU CUGCUCAAACUGGAAUUGCCGUUUUAGAUAUGUGUGCU UCAUUAAAAGAAUUACUGCAAAAUGGUAUGAAUGGACG UACCAUAUUGGGUAGUGCUUUAUUAGAAGAUGAAUUUA CACCUUUUGAUGUUGUUAGACAAUGCUCAGGUGUUACU UUCCAA |
| 2 | 5'-UTR | GGGAAAUAAGAGAGAAAGAAGAGUAAGAAGAAAUAUA AGACCCCGGCGCC |
| 3 | Kozak sequence | GCCACC |

(continued)

| SEQ ID NO: | Description of sequences | Sequence information |
|---|---|---|
| 4 | 3'-UTR | GCUGGAGCCUCGGUGGCCUAGCUUCUUGCCCCUUGGGCC UCCCCCCAGCCCCUCCUCCCCUUCCUGCACCCGUACCCCC GUGGUCUUUGAAUAAAGUCUGAGUGGGCGGCA |
| 5 | Poly-A tail | AAAAAAAAAAAAAAAAAAAAAAAAAAAAAAAAGCAUAUGA CUAAAAAAAAAAAAAAAAAAAAAAAAAAAAAAAAAAAAAA AAAAAAAAAAAAAAAAAAAAAAAAAAAAAAAAAA |
| 6 | Amino acid sequences of 3CL protease of SARS-CoV-2 | MSGFRKMAFPSGKVEGCMVQVTCGTTTLNGLWLDDVVYCP RHVICTSEDMLNPNYEDLLIRKSNHNFLVQAGNVQLRVIGHS MQNCVLKLKVDTANPKTPKYKFVRIQPGQTFSVLACYNGSP SGVYQCAMRPNFTIKGSFLNGSCGSVGFNIDYDCVSFCYMH HMELPTGVHAGTDLEGNFYGPFVDRQTAQAAGTDTTITVNV LAWLYAAVINGDRWFLNRFTTTLNDFNLVAMKYNYEPLTQD HVDILGPLSAQTGIAVLDMCASLKELLQNGMNGRTILGSALL EDEFTPFDVVRQCSGVTFQ |

(continued)

| SEQ ID NO: | Description of sequences | Sequence information |
|---|---|---|
| 7 | Complete nucleotide sequences comprising mRNA encoding 3CL protease of SARS-CoV-2 | GGGAAAUAAGAGAGAAAAGAAGAGUAAGAAGAAAUAUA AGACCCCGGCGCC<br>GCCACC<br>AUG<br>AGUGGUUUUAGAAAAAUGGCAUUCCCAUCUGGUAAAGU UGAGGGUUGUAUGGUACAAGUAACUUGUGGUACAACUA CACUUAACGGUCUUUGGCUUGAUGACGUAGUUUACUGU CCAAGACAUGUGAUCUGCACCUCUGAAGACAUGCUUAA CCCUAAUUAUGAAGAUUUACUCAUUCGUAAGUCUAAUC AUAAUUUCUUGGUACAGGCUGGUAAUGUUCAACUCAGG GUUAUUGGACAUUCUAUGCAAAAUUGUGUACUUAAGCU UAAGGUUGAUACAGCCAAUCCUAAGACACCUAAGUAUA AGUUUGUUCGCAUUCAACCAGGACAGACUUUUUCAGUG UUAGCUUGUUACAAUGGUUCACCAUCUGGUGUUUACCA AUGUGCUAUGAGGCCCAAUUUCACUAUUAAGGGUUCAU UCCUUAAUGGUUCAUGUGGUAGUGUUGGUUUUAACAUA GAUUAUGACUGUGUCUCUUUUUGUUACAUGCACCAUAU GGAAUUACCAACUGGAGUUCAUGCUGGCACAGACUUAG AAGGUAACUUUUAUGGACCUUUUGUUGACAGGCAAACA GCACAAGCAGCUGGUACGGACACAACUAUUACAGUUAA UGUUUUAGCUUGGUUGUACGCUGCUGUUAUAAAUGGAG ACAGGUGGUUUCUCAAUCGAUUUACCACAACUCUUAAU GACUUUAACCUUGUGGCUAUGAAGUACAAUUAUGAACC UCUAACACAAGACCAUGUUGACAUACUAGGACCUCUUU CUGCUCAAACUGGAAUUGCCGUUUUAGAUAUGUGUGCU UCAUUAAAAGAAUUACUGCAAAAUGGUAUGAAUGGACG UACCAUAUUGGGUAGUGCUUUAUUAGAAGAUGAAUUUA CACCUUUUGAUGUUGUUAGACAAUGCUCAGGUGUUACU UUCCAA<br>UGAUAAUAG<br>GCUGGAGCCUCGGUGGCCUAGCUUCUUGCCCCUUGGGCC UCCCCCCAGCCCCUCCUCCCCUUCCUGCACCCGUACCCCC GUGGUCUUUGAAUAAAGUCUGAGUGGGCGGCA<br>AAAAAAAAAAAAAAAAAAAAAAAAAAAAAGCAUAUGA CUAAAAAAAAAAAAAAAAAAAAAAAAAAAAAAAAAAA AAAAAAAAAAAAAAAAAAAAAAAAAAAAAA |

(continued)

| SEQ ID NO: | Description of sequences | Sequence information |
|---|---|---|
| 8 | Nucleotide sequences encoding 3CL protease of SARS-CoV | AGGAAAAUGGCAUUCCCGUCAGGCAAAGUUGAAGGGUG CAUGGUACAAGUAACCUGUGGAACUACAACUCUUAAUG GAUUGUGGUUGGAUGACACAGUAUACUGUCCAAGACAU GUCAUUUGCACAGCAGAAGACAUGCUUAAUCCUAACUA UGAAGAUCUGCUCAUUCGCAAAUCCAACCAUAGCUUUC UUGUUCAGGCUGGCAAUGUUCAACUUCGUGUUAUUGGC CAUUCUAUGCAAAAUUGUCUGCUUAGGCUUAAAGUUGA UACUUCUAACCCUAAGACACCCAAGUAUAAAUUUGUCC GUAUCCAACCUGGUCAAACAUUUUCAGUUCUAGCAUGC UACAAUGGUUCACCAUCUGGUGUUUAUCAGUGUGCCAU GAGACCUAAUCAUACCAUUAAAGGUUCUUUCCUUAAUG GAUCAUGUGGUAGUGUUGGUUUUAACAUUGAUUAUGAU UGCGUGUCUUUCUGCUAUAUGCAUCAUAUGGAGCUUCC AACAGGAGUACACGCUGGUACUGACUUAGAAGGUAAAU UCUAUGGUCCAUUUGUUGACAGACAAACUGCACAGGCU GCAGGUACAGACACAACCAUAACCUUAAAUGUUUUGGC AUGGCUGUAUGCUGCUGUUAUCAAUGGUGAUAGGUGGU UUCUUAAUAGAUUCACCACUACUUUGAAUGACUUUAAC CUUGUGGCAAUGAAGUACAACUAUGAACCUUUGACACA AGAUCAUGUUGACAUAUUGGGACCUCUUUCUGCUCAAA CAGGAAUUGCCGUCUUAGAUAUGUGUGCUGCUUUGAAA GAGCUGCUGCAGAAUGGUAUGAAUGGUCGUACUAUCCU UGGUAGCACUAUUUUAGAAGAUGAGUUUACACCAUUUG AUGUUGUUAGACAAUGC |

(continued)

| SEQ ID NO: | Description of sequences | Sequence information |
|---|---|---|
| 9 | Complete nucleotide sequences comprising mRNA encoding 3CL protease of SARS-CoV | GGGAAAUAAGAGAGAAAAGAAGAGUAAGAAGAAAUAUA AGACCCCGGCGCC<br>GCCACC<br>AUG<br>AGGAAAAUGGCAUUCCCGUCAGGCAAAGUUGAAGGGUG CAUGGUACAAGUAACCUGUGGAACUACAACUCUUAAUG GAUUGUGGUUGGAUGACACAGUAUACUGUCCAAGACAU GUCAUUUGCACAGCAGAAGACAUGCUUAAUCCUAACUA UGAAGAUCUGCUCAUUCGCAAAUCCAACCAUAGCUUUC UUGUUCAGGCUGGCAAUGUUCAACUUCGUGUUAUUGGC CAUUCUAUGCAAAAUUGUCUGCUUAGGCUUAAAGUUGA UACUUCUAACCCUAAGACACCCAAGUAUAAAUUUGUCC GUAUCCAACCUGGUCAAACAUUUUCAGUUCUAGCAUGC UACAAUGGUUCACCAUCUGGUGUUUAUCAGUGUGCCAU GAGACCUAAUCAUACCAUUAAAGGUUCUUUCCUUAAUG GAUCAUGUGGUAGUGUUGGUUUUAACAUUGAUUAUGAU UGCGUGUCUUUCUGCUAUAUGCAUCAUAUGGAGCUUCC AACAGGAGUACACGCUGGUACUGACUUAGAAGGUAAAU UCUAUGGUCCAUUUGUUGACAGACAAACUGCACAGGCU GCAGGUACAGACACAACCAUAACCUUAAAUGUUUUGGC AUGGCUGUAUGCUGCUGUUAUCAAUGGUGAUAGGUGGU UUCUUAAUAGAUUCACCACUACUUUGAAUGACUUUAAC CUUGUGGCAAUGAAGUACAACUAUGAACCUUUGACACA AGAUCAUGUUGACAUAUUGGGACCUCUUUCUGCUCAAA CAGGAAUUGCCGUCUUAGAUAUGUGUGCUGCUUUGAAA GAGCUGCUGCAGAAUGGUAUGAAUGGUCGUACUAUCCU UGGUAGCACUAUUUUAGAAGAUGAGUUUACACCAUUUG AUGUUGUUAGACAAUGC<br>UGAUAAUAG<br>GCUGGAGCCUCGGUGGCCUAGCUUCUUGCCCCUUGGGCC UCCCCCCAGCCCCUCCUCCCCUUCCUGCACCCGUACCCCC GUGGUCUUUGAAUAAAGUCUGAGUGGGCGGCA AAAAAAAAAAAAAAAAAAAAAAAAAAAAAAGCAUAUGA CUAAAAAAAAAAAAAAAAAAAAAAAAAAAAAAAAAAAA AAAAAAAAAAAAAAAAAAAAAAAAAAAAAAAA |
| 10 | Amino acid sequences of 3CL protease of SARS-CoV | MRKMAFPSGKVEGCMVQVTCGTTTLNGLWLDDTVYCPRH VICTAEDMLNPNYEDLLIRKSNHSFLVQAGNVQLRVIGHSMQ NCLLRLKVDTSNPKTPKYKFVRIQPGQTFSVLACYNGSPSGV YQCAMRPNHTIKGSFLNGSCGSVGFNIDYDCVSFCYMHHME LPTGVHAGTDLEGKFYGPFVDRQTAQAAGTDTTITLNVLAW LYAAVINGDRWFLNRFTTTLNDFNLVAMKYNYEPLTQDHVDI LGPLSAQTGIAVLDMCAALKELLQNGMNGRTILGSTILEDEF TPFDVVRQC |

(continued)

| SEQ ID NO: | Description of sequences | Sequence information |
|---|---|---|
| 11 | Nucleotide sequences encoding 3CL protease of MERS-CoV | GUGAAAAUGUCACAUCCCAGUGGAGAUGUUGAGGCUUG UAUGGUUCAGGUUACCUGCGGUAGCAUGACUCUUAAUG GUCUUUGGCUUGACAACACAGUCUGGUGCCCACGACAC GUAAUGUGCCCGGCUGACCAGUUGUCUGAUCCUAAUUA UGAUGCCUUGUUGAUUUCUAUGACUAAUCAUAGUUUCA GUGUGCAAAAACACAUUGGCGCUCCAGCAAACUUGCGU GUUGUUGGUCAUGCCAUGCAAGGCACUCUUUUGAAGUU GACUGUCGAUGUUGCUAACCCUAGCACUCCAGCCUACAC UUUUACAACAGUGAAACCUGGCGCAGCAUUUAGUGUGU UAGCAUGCUAUAAUGGUCGUCCGACUGGUACAUUCACU GUUGUAAUGCGCCCUAACUACACAAUUAAGGGUUCCUU UCUGUGUGGUUCUUGUGGUAGUGUUGGUUACACCAAGG GGGGUAGUGUGAUCAAUUUUUGUUACAUGCAUCAAAUG GAACUUGCUAAUGGUACACAUACCGGUUCAGCAUUUGA UGGUACUAUGUAUGGUGCCUUUAUGGAUAAACAAGUGC ACCAAGUUCAGUUAACAGACAAAUACUGCAGUGUUAAU GUAGUAGCUUGGCUUUACGCAGCAAUACUUAAUGGUUG CGCUUGGUUUGUAAAACCUAAUCGCACUAGUGUUGUUU CUUUUAAUGAAUGGGCUCUUGCCAACCAAUUCACUGAA UUUGUUGGCACUCAAUCCGUUGACAUGUUAGCUGUCAA AACAGGCGUUGCUAUUGAACAGCUGCUUUAUGCGAUCC AACAACUUUAUACUGGGUUCCAGGGAAAGCAAAUCCUU GGCAGUACCAUGUUGGAAGAUGAAUUCACACCUGAGGA UGUUAAUAUGCAGAUU |

(continued)

| SEQ ID NO: | Description of sequences | Sequence information |
|---|---|---|
| 12 | Complete nucleotide sequences comprising mRNA encoding 3CL protease of MERS-CoV | GGGAAAUAAGAGAGAAAAGAAGAGUAAGAAGAAAUAUA AGACCCCGGCGCC<br>GCCACC<br>AUG<br>GUGAAAAUGUCACAUCCCAGUGGAGAUGUUGAGGCUUG UAUGGUUCAGGUUACCUGCGGUAGCAUGACUCUUAAUG GUCUUUGGCUUGACAACACAGUCUGGUGCCCACGACAC GUAAUGUGCCCGGCUGACCAGUUGUCUGAUCCUAAUUA UGAUGCCUUGUUGAUUUCUAUGACUAAUCAUAGUUUCA GUGUGCAAAAACACAUUGGCGCUCCAGCAAACUUGCGU GUUGUUGGUCAUGCCAUGCAAGGCACUCUUUUGAAGUU GACUGUCGAUGUUGCUAACCCUAGCACUCCAGCCUACAC UUUUACAACAGUGAAACCUGGCGCAGCAUUUAGUGUGU UAGCAUGCUAUAAUGGUCGUCCGACUGGUACAUUCACU GUUGUAAUGCGCCCUAACUACACAAUUAAGGGUUCCUU UCUGUGUGGUUCUUGUGGUAGUGUUGGUUACACCAAGG GGGGUAGUGUGAUCAAUUUUUGUUACAUGCAUCAAAUG GAACUUGCUAAUGGUACACAUACCGGUUCAGCAUUUGA UGGUACUAUGUAUGGUGCCUUUAUGGAUAAACAAGUGC ACCAAGUUCAGUUAACAGACAAAUACUGCAGUGUUAAU GUAGUAGCUUGGCUUUACGCAGCAAUACUUAAUGGUUG CGCUUGGUUUGUAAAACCUAAUCGCACUAGUGUUGUUU CUUUUAAUGAAUGGGCUCUUGCCAACCAAUUCACUGAA UUUGUUGGCACUCAAUCCGUUGACAUGUUAGCUGUCAA AACAGGCGUUGCUAUUGAACAGCUGCUUUAUGCGAUCC AACAACUUUAUACUGGGUUCCAGGGAAAGCAAAUCCUU GGCAGUACCAUGUUGGAAGAUGAAUUCACACCUGAGGA UGUUAAUAUGCAGAUU<br>UGAUAAUAG<br>GCUGGAGCCUCGGUGGCCUAGCUUCUUGCCCCUUGGGCC UCCCCCCAGCCCCUCCUCCCCUUCCUGCACCCGUACCCCC GUGGUCUUUGAUAAAGUCUGAGUGGGCGGCA AAAAAAAAAAAAAAAAAAAAAAAAAAAAAAGCAUAUGA CUAAAAAAAAAAAAAAAAAAAAAAAAAAAAAAAAAAAA AAAAAAAAAAAAAAAAAAAAAAAAAAAAAAAA |
| 13 | Amino acid sequences of 3CL protease of MERS-CoV | MVKMSHPSGDVEACMVQVTCGSMTLNGLWLDNTVWCPRH VMCPADQLSDPNYDALLISMTNHSFSVQKHIGAPANLRVVG HAMQGTLLKLTVDVANPSTPAYTFTTVKPGAAFSVLACYNG RPTGTFTVVMRPNYTIKGSFLCGSCGSVGYTKGGSVINFCYM HQMELANGTHTGSAFDGTMYGAFMDKQVHQVQLTDKYCS VNVVAWLYAAILNGCAWFVKPNRTSVVSFNEWALANQFTEF VGTQSVDMLAVKTGVAIEQLLYAIQQLYTGFQGKQILGSTML<br><br>EDEFTPEDVNMQI |

Note: according to WIPO Standard ST. 26, the symbol "u" shall not be used to represent uracil in an RNA molecule in the sequence listing, and the symbol "t" in the RNA sequence is understood to mean the uracil.

**Detailed Description of the Preferred Embodiment**

The present application is now described with reference to the following examples which are intended to illustrate the present application (but not to limit the present application). The following examples may include a compilation of the data collected at various times during development and experimentation relating to the subject matter of the present disclosure.

Unless otherwise specified, the molecular biology experimental methods and immunoassays used in the present application were generally carried out in accordance with the methods described in Molecular Cloning: A Laboratory Manual, 2nd Edition, Cold Spring Harbor Laboratory Press, 1989, by J. Sambrook et al., and Short Protocols in Molecular Biology, 3rd Edition, John Wiley & Sons, Inc., 1995, by F. M. Ausubel et al.; the use of restriction endonucleases was in accordance with the conditions recommended by the product manufacturer. All reagents or instruments used of which the manufacturers are not given are commercially available conventional products. Those skilled in the art will understand that the examples describe the present application by way of illustration only and are not intended to limit the scope of the claimed subject matter of the present application. All publications and other references mentioned herein are incorporated by reference in their entirety.

**Example 1 In Vitro Transcription Synthesis of mRNA Encoding 3CL Protease of SARS-CoV-2**

Experimental Materials: as used in the experiments, the pUC57 vector was purchased from Addgene (Cat. No.: 54338), ATP was purchased from Thermo (Cat. No.: R0441), CTP was purchased from Thermo (Cat. No.: 18331017), GTP was purchased from Thermo (Cat. No.: 18332015), UTP was purchased from Thermo (Cat. No.: R1471), Nuclease-free water was purchased from Thermo (Cat. No.: 10977015), Top10 competence cells were purchased from Invitrogen (Cat. No.: C404010), gentamicin was purchased from Sangon Biotech Co., Ltd. (Cat. No.: A100304), primers were synthesized by Sangon Biotech Co., Ltd., AccuPrime™ Taq High-Fidelity DNA Polymerase was purchased from Invitrogen (Cat. No.: 12346086), Endotoxin-Free Plasmid Extraction Kit was purchased from Invitrogen (Cat. No.: K210004), T7 RNA polymerase was purchased from Thermo (Cat. No.: EP0111), Inorganic Pyrophosphatase was purchased from Thermo (Cat. No.: EF0221), RNase inhibitor was purchased from Applied Biosystems (Cat. No.: N8080119), modified nucleotide (N1-methylpseudouridine) was purchased from Yeasen Biotechnology (Cat. No.: 10651ES80), S-adenosyl methionine (SAM) was purchased from NEN (Cat. No.: B9003S), Cap analogue (m7G(5')ppp(5')G) was purchased from Invitrogen (Cat. No.: AM8050), and DNase I was purchased from Thermo (Cat. No.:18047019). The gene amplification instrument was purchased from Hangzhou Bio-Gener Technology Co., Ltd. (Model No. AUTO-96), the Nanodrop Ultra Microvolume UV-Vis Spectrophotometer was purchased from Thermo (Cat. No.:840-317400), the thermostatic mixer (refrigerated) was purchased from Kylin-Bell Lab Instruments (Cat. No.: BE-3200), the refrigerated centrifuge was purchased from Germany/Eppendorf (Model: 5424R), and the dry bath incubator was purchased from Hangzhou Allsheng Instruments Co., Ltd. (model: K30).

**Step** I: **Generation of a DNA Template**

The mRNA template sequence was designed by comprehensively considering codon bias, GC content, thermodynamic stability of RNA secondary structure, and the like, and an in vitro transcription template plasmid with pUC57 as the backbone was constructed by de novo gene synthesis. The pUC57 vector template contained a T7 promoter, a 5'UTR, a Kozak, and a 3'UTR sequence, and the encoding region of 3CL protease of SARS-CoV-2 (with an amino acid sequence as set forth in SEQ ID NO: 6) was inserted between the UTRs. The synthesized plasmid was transformed into host bacteria and spread onto a solid plate containing antibiotics, positive clones were verified by colony PCR and Sanger sequencing, and by making alignment, the plasmid sequencing file was confirmed to be the gene sequence of interest; the correct clones were amplified in a liquid medium and the plasmids were purified by using an endotoxin-free plasmid extraction kit. DNA tailing and linearization of the plasmid template were performed by PCR amplification using a PCR primer carrying PolyA_110 and a high-fidelity DNA polymerase. The PCR product was purified to remove enzymes and reaction components to obtain the linearized template. The concentration of the product and the A260/280 ratio were determined by Nanodrop, and the quality of the product was detected by agarose gel electrophoresis.

**Step II** *In Vitro* **Transcription of mRNA**

*In vitro* co-transcription was carried out by using T7 RNA polymerase, inorganic pyrophosphatase, RNase inhibitor, nucleotide, modified nucleotide (N1-methylpseudouridine), SAM, cap analogue, and linearized template, to generate the capped mRNA in one step. The DNA template in the product was digested with DNase I. The transcription product was purified by column chromatography to obtain the mRNA of interest. The concentration of the product and the A260/280 ratio were measured by Nanodrop, and the quality of the product was detected by denatured agarose gel electrophoresis, and the final mRNA product of interest was obtained. The mRNA was stored in RNase-free water, with a purity of ≥90%, at - 80 °C. The nucleotide sequence of the finally obtained mRNA molecule was as set forth in SEQ ID NO: 7, wherein the sequence encoding the 3CL protease of SARS-CoV-2 was as set forth in SEQ ID NO: 1.

By referring to the above method, the mRNA encoding 3CL protease of SARS-CoV and the mRNA encoding 3CL protease of MERS-CoV are further synthesized by *in vitro* transcription, with the sequences as set forth in SEQ ID NO: 9 and SEQ ID NO: 12, respectively.

**Example 2 Effects of mRNA encoding 3CL Protease of SARS-CoV-2 on the Growth Viability of various cell lines**

The example was performed according to the mRNA encoding 3CL Protease of SARS-CoV-2 prepared in Example 1.

Experimental Materials: tumor cell lines used in the experiments included, for example, human glioblastoma cell line U87 MG purchased from ATCC (Cat. No.: HTB-14), A172 purchased from ATCC (Cat. No.: CRL-1620), human osteosarcoma cell line U2OS purchased from ATCC (Cat. No.: HTB-96), human melanoma cell line CHL-1 purchased from ATCC (Cat. No.: CRL-9446), human prostatic cancer cell line DU145 purchased from ATCC (Cat. No.: HTB-81), human colon cancer cell line HCT-116 purchased from ATCC (Cat. No.: CCL-247), human cervical cancer cell line Hela purchased from ATCC (Cat. No.: CCL-2), human gastric adenocarcinoma cell line AGS purchased from ATCC (Cat. No.: CRL-1739), human lung cancer cell line A549 purchased from ATCC (Cat. No.: CCL-185), human non-small cell lung cancer cell line NCI-H1975 purchased from ATCC (Cat. No.: CRL-5908), human bronchoalveolar carcinoma cell line NCI-H1650 purchased from ATCC (Cat. No.: CRL-5883), human renal cancer cell line ACHN purchased from ATCC (Cat. No.: CRL-1611), human liver cancer cell line Huh7 purchased from Chinese National Infrastructure of Cell Line Resource, human pancreatic cancer cell line PANC-1 purchased from ATCC (Cat. No.: CRL-1469). Non-tumor cell lines used in the test included, for example, human venous endothelial cell line HUVEC purchased from Zhejiang Meisen Cell Technology Co., Ltd. (Cat. No.: CTCC-0804-PC), and human embryonic kidney epithelial cell 293T purchased from ATCC (Cat. No.: CRL-3216).

The above tumor cell lines were cultured in a cell incubator at 37°C with 5% $CO_2$. The complete medium used for cell growth was a DMEM medium with high glucose (purchased from Gibco, Cat. No.: 11995065) containing 10% fetal bovine serum (purchased from Gibco, Cat. No.: 16000044) and a penicillin-streptomycin double antibiotics (purchased from Gibco, Cat. No.: 2321152), an MEM medium (purchased from Gibco, Cat. No.: 10370021), a McCoy's 5A medium (purchased from Gibco, Cat. No.: 16600082), an F-12K medium (purchased from Gibco, Cat. No.: 21127022) or a RPMI-1640 medium (purchased from Gibco, Cat. No.: 22400089).

The complete medium used for HUVEC cells was a endothelial cell-specific medium containing 10% fetal bovine serum and penicillin-streptomycin double antibiotics, purchased from Zhejiang Meisen Cell Technology Co., Ltd. (Cat. No.: M22SN3004).

The complete culture medium used for 293T cells was a DMEM medium with high glucose (purchased from Gibco, Cat. No.: 11995065) containing 10% fetal bovine serum (purchased from Gibco, Cat. No.: 16000044) and penicillin-streptomycin double antibiotics (purchased from Gibco, Cat. No.: 2321152).

The Cell-Titer Glo® Luminescent Cell Viability Assay Solution was purchased from Promega (Cat. No.: G7572). The microplate reader was purchased from Molecular Devices, Model No. SpectraMax M5.

The experimental scheme was as follows:

The test cells were seeded in a white-walled clear-bottom 96-well plate at a density of about $1.0 \times 10^4$ per well and cultured at 37 °C with 5% $CO_2$ for 24 hours. Subsequently, the mRNA encoding 3CL Protease of SARS-CoV-2 was diluted gradually with Opti-MEM and transfected into cells using Lipo2000 liposomes. The final concentrations of the mRNA encoding 3CL Protease of SARS-CoV-2 transfected into 293T cells and HUVEC cells were: 10000 ng/ml, 5000 ng/ml, 2500 ng/ml, 1250 ng/ml, 625 ng/ml, 312.5ng/ml, 156.25ng/ml, 78.125ng/ml, and 39.06 ng/ml. The final concentrations of the mRNA transfected into ACHN cells, Hela cells, U2OS cells, DU145 cells, U87 MG cells, A172 cells, A549 cells, NCI-H1975 cells, and NCI-H1650 cells were 1000 ng/ml, 500 ng/ml, 250 ng/ml, 125ng/ml, 62.5 ng/ml, 31.25 ng/ml, and 15.65 ng/ml. The final concentrations of the mRNA transfected into PANC-1 cells, AGS cells, Huh7 cells, CHL-1 cells, and HCT-116 cells were 2000 ng/ml, 1000 ng/ml, 500 ng/ml, 250 ng/ml, 125ng/ml, 62.5 ng/ml, 31.25 ng/ml. The final concentration of the mRNA transfected into the control group was 0, and the other conditions were completely identical to those of the test group. After 48 h, the supernatant was discarded, and 100 $\mu$l of the diluted Cell-Titer Glo® Luminescent Cell Viability Assay solution were added. The mixture was shaken for 5 minutes in the dark to lyse the cells, followed by standing for 3 minutes. Finally, the luminescence value of each well was measured using a microplate reader.

Calculation formula: Cell viability (%) = average value of test group/average value of cell control group $\times$ 100

Statistical analysis: The inhibition rate-concentration curve was fitted to a sigmoidal curve using Origin 9.0 software to calculate the half maximal effective concentration ($EC_{50}$) of EV-3C-mRNA on the cells.

The test results were shown in Tables 1 to 2 and Fig. 1, and Fig. 1 showed the sigmoidal curves and $IC_{50}$ values of the cell viability corresponding to Tables 1 and 2.

Table 1 Effects of mRNA encoding 3CL Protease of SARS-CoV-2 on the viability of non-tumor cell lines

| Concentration (ng/ml) | 0 | 39.06 | 78.125 | 156.25 | 312.5 | 625 | 1250 | 2500 | 5000 | 10000 |
|---|---|---|---|---|---|---|---|---|---|---|
| Cell viability of 293T (%) | 99.99± 7.57 | 108.86 ±4.96 | 109.35 ±5.13 | 108.12 ±5.44 | 105.29 ±6.57 | 102.02 ±2.83 | 104.56 ±2.89 | 106.61 ±1.35 | 102.42 ±1.36 | 101.84 ±4.65 |
| Cell viability of HUVEC (%) | 99.99± 7.86 | 61.02± 16.40 | 66.81± 5.97 | 60.64± 9.13 | 65.06± 6.47 | 59.33± 1.77 | 57.24± 7.52 | 53.94± 14.71 | 51.31± 8.88 | / |

Table 1 listed the effects of the mRNA encoding 3CL Protease of SARS-CoV-2 at different concentrations on the viability of two human non-tumor cell lines. It was seen from the sigmoidal curves shown in panels A and B of Fig. 1 that the mRNA had no significant inhibitory effect on the viability of the two normal cell lines. Specifically, as shown in panel A of Fig. 1, the $IC_{50}$ value of the mRNA encoding 3CL Protease of SARS-CoV-2 against normal human embryonic kidney cell lines 293 T was greater than 10000 ng/ml; as shown in panel B of Fig. 1, the $IC_{50}$ value against human umbilical vein endothelial cell lines HUVEC was greater than 5000 ng/ml. The above results indicated that the mRNA provided by the present application had no significant toxic effect on the two normal cell lines.

Table 2 Effects of mRNA encoding 3CL Protease of SARS-CoV-2 on the viability of tumor cell lines

| Concentration (ng/ml) | 0 | 31.25 | 62.5 | 125 | 250 | 500 | 1000 | 2000 |
|---|---|---|---|---|---|---|---|---|
| Cell viability of PANC-1 (%) | 100±2.3 1 | 91.24±0.7 0 | 87.48±3. 66 | 79.41±5.0 6 | 77.46±0.5 2 | 48.36±3. 44 | 40.02±1 .05 | 21.14±6. 94 |
| Cell viability of AGS (%) | 100±4.6 9 | 96.17±3.0 5 | 75.11±0. 85 | 65.88±2.0 8 | 59.93±3.8 8 | 21.54±0. 84 | 22.02±0 .24 | 8.68±3.8 1 |
| Cell viability of Huh7 (%) | 100±0.6 7 | 89.58±0.5 9 | 80.4±2. 86 | 62.52±4.3 6 | 62.96+2.9 1 | 30.13+1. 69 | 28.04+1 .46 | 20.37±5. 19 |
| Cell viability of CHL-1 (%) | 100±0.1 6 | 86.16±0.1 5 | 82.30±2. 01 | 59.25±8.4 2 | 67.52±0.0 7 | 44.99±5. 17 | 41.04±0 .87 | 24.14±7. 97 |
| Cell viability of HCT-116 (%) | 100±0.4 2 | 95.75±0.4 2 | 91.11±1. 99 | 79.58±2.1 9 | 86.28±7.0 7 | 67.30±3. 31 | 61.36±1 .29 | 46.57±8. 39 |
| Concentration (ng/ml) | 0 | 15.65 | 31.25 | 62.5 | 125 | 250 | 500 | 1000 |
| Cell viability of ACHN (%) | 100±4.2 7 | 120.52±1 0.65 | 114.57± 8.03 | 108.60±1 0.78 | 108.32±1 2.35 | 45.57±1 3.91 | 39.97±1 4.4 | 73.22±1 2.88 |
| Cell viability of Hela (%) | 100±7.8 4 | 86.35±11. 35 | 88.93±1 3.39 | 64.16±3.0 2 | 69.83±17. 36 | 46.75±3. 91 | 40.89±3 .39 | 26.21±3. 67 |
| Cell viability of U2OS (%) | 100±5.9 3 | 87.14±3.3 8 | 84.79±7. 40 | 68.85±2.8 1 | 65.85±10. 35 | 31.27±6. 55 | 6.94±1. 40 | 2.07±0.6 7 |
| Cell viability of DU145 (%) | 100±2.4 5 | 85.28±4.6 8 | 82.31±6. 77 | 47.48±6.4 4 | 33.94±3.3 2 | 25.91±6. 07 | 12.61±1 .41 | 3.66±1.3 5 |
| Cell viability of U87 MG (%) | 100±4.4 6 | 115.48±5. 10 | 111.47± 4.65 | 81.36±2.8 8 | 73.98±10. 32 | 39.80±6. 57 | 9.51±0. 54 | 5.17±0.7 2 |
| Cell viability of A172 (%) | 99.99±3 .23 | 100.43±2. 25 | 91.43±5. 37 | 79.10±4.0 4 | 57.33±9.4 9 | 33.10±6. 84 | 17.64±4 .6 | 20.54±3. 51 |
| Cell viability of A549 (%) | 99.99±6 .57 | 86.29±2.0 2 | 79.86±6. 04 | 62.05±10. 99 | 39.43±8.6 9 | 26.21±2. 38 | 15.31±2 .28 | 22.71±1. 93 |
| Cell viability of NCI-H1975 (%) | 100±3.3 7 | 108.11±4. 67 | 104.95± 5.19 | 95.50±5.9 1 | 73.95±16. 27 | 39.38±8. 25 | 31.12±5 .25 | 21.44±4. 62 |
| Cell viability of NCI-H1650 (%) | 100±2.8 6 | 84.66±4.5 2 | 8.35±3.1 6 | 68.54±2.7 3 | 54.91±9.0 1 | 34.04±6. 91 | 28.01±4 .82 | 25.98±3. 03 |

Table 2 listed the effects of the mRNA encoding 3CL Protease of SARS-CoV-2 at different concentrations on the viability of various human tumor cell lines. It was seen from the sigmoidal curves shown in panels C to P of Fig. 1 that the mRNA provided by the present application had significant killing effects on the growth of various tumor cell lines. Specifically, as shown in panel C of Fig. 1, the $IC_{50}$ value of the mRNA against human pancreatic cancer cell line PANC-1 was $574.626 \pm 52.61$ ng/ml; as shown in panel D of Fig. 1, the $IC_{50}$ value against human glioblastoma cell line U87 was $184.268 \pm 25.76$ ng/ml; as shown in panel E of Fig. 1, the $IC_{50}$ value against human glioblastoma cell line A172 was $175.569 \pm 35.83$ ng/ml; as shown in panel F of Fig. 1, the $IC_{50}$ value against human gastric adenocarcinoma cancer cell line AGS was $248.778 \pm 14.17$ ng/ml; as shown in panel G of Fig. 1, the $IC_{50}$ value against human liver cancer cell line Huh7 was $310.065 \pm 20.67$ ng/ml; as shown in panel H of Fig. 1, the $IC_{50}$ value against human melanoma cell line CHL-1 was $444.233 \pm 57.54$ ng/ml; as shown in panel I of Fig. 1, the $IC_{50}$ value against human colon cancer cell HCT116 was $976.261 \pm 262.17$ ng/ml; as shown in panel J of Fig. 1, the $IC_{50}$ value against human renal cancer cell line ACHN was $430.272 \pm 107.46$ ng/ml; as shown in panel K of Fig. 1, the $IC_{50}$ value against human cervical cancer cell line Hela was $258.346 \pm 20.20$ ng/ml; as shown in panel L of Fig. 1, the $IC_{50}$ value against human osteosarcoma cell line U2OS was $122.175 \pm 14.78$ ng/ml; as shown in panel M of Fig. 1, the $IC_{50}$ value against human prostatic cancer cell line DU145 was $78.716 \pm 8.20$ ng/ml; as shown in panel N of Fig. 1, the $IC_{50}$ value against human lung cancer cell line NCI-H1975 was $274.938 \pm 61.75$ ng/ml; as shown in panel O of Fig. 1, the $IC_{50}$ value against human lung cancer cell line NCI-H1650 was $158.56 \pm 42.74$ ng/ml; as shown in panel P of Fig. 1, the $IC_{50}$ value against human lung cancer cell line A549 was $106.69 \pm 24.75$ ng/ml. In summary, the $IC_{50}$ values of the test mRNA against all test human tumor cell lines each were less than 1000 ng/ml, significantly lower than the $IC_{50}$ value of the mRNA against the four non-tumor cell lines, indicating that the mRNA provided by the present application had broad-spectrum anti-tumor activity and good selectivity.

Table 3 Therapeutic Indexes of mRNA encoding 3CL Protease of SARS-CoV-2

| Name of cell lines | Types of cell | $IC_{50}$ (ng/mL) | Therapeutic index |
|---|---|---|---|
| 293T | Normal cells | >10000 | - |
| PANC-1 | Pancreatic cancer | $574.626 \pm 52.61$ | >17.4 |
| Huh7 | Liver cancer | $310.07 \pm 20.67$ | >32.25 |
| HCT-116 | Colon cancer | $976.261 \pm 262.17$ | >10.24 |
| AGS | Gastric adenocarcinoma | $248.778 \pm 14.17$ | >40.19 |
| ACHN | Renal cancer | $430.272 \pm 107.46$ | >23.24 |
| CHL-1 | Melanoma | $444.233 \pm 57.54$ | >22.51 |
| Hela | Cervical cancer | $258.346 \pm 20.20$ | >38.70 |
| U2OS | Osteosarcoma | $122.175 \pm 14.78$ | >81.84 |
| DU145 | Prostatic cancer | $78.716 \pm 8.20$ | >127.03 |
| U87 | Glioblastoma | $184.268 \pm 25.76$ | >54.26 |
| A172 | Glioblastoma | $175.569 \pm 35.83$ | >56.95 |
| A549 | Lung cancer | $106.69 \pm 25.75$ | >93.72 |
| NCI-H1975 | Non-small cell lung Adenocarcinoma | $274.938 \pm 61.75$ | >36.37 |
| NCI-H1650 | Lung Bronchoalveolar carcinoma | $158.56 \pm 42.74$ | >63.06 |

Table 3 showed the therapeutic index of the mRNA encoding 3CL Protease of SARS-CoV-2 against various tumor cell line, with the 293T cell as the control cell. The formula is as follows: Therapeutic index = Maximal $IC_{50}$ value of 293T cell ÷ $IC_{50}$ value of each tumor cell line. The results in the Table showed that the mRNA encoding 3CL Protease of SARS-CoV-2 provided by the present application exhibited good therapeutic safety.

By referring to the test method of Example 2, the effects of the mRNA encoding 3CL Protease of SARS-CoV and mRNA encoding 3CL Protease of MERS-CoV on the viability of various cell lines, where the kinds of the test cell line were the same as those in Example 2, and the results showed that the two mRNAs also exhibited broad-spectrum anti-tumor activity and good selectivity.

The basic principles, main features and advantages of the present application have been shown and described above. It should be understood by those skilled in the art that the above examples do not limit the present application in any way, and all technical solutions obtained by means of equivalent substitutions or equivalent changes fall within the protection scope of the present application.

**Claims**

1. Use of a 3CL protease of coronavirus or a nucleic acid molecule encoding the 3CL protease of coronavirus in the preparation of a medicament for the prevention and/or treatment of a tumor.

2. The use according to claim 1, wherein the coronavirus is SARS-CoV, MERS-CoV or SARS-CoV-2, preferably SARS-CoV-2.

3. The use according to claim 1 or 2, wherein the 3CL protease has an amino acid sequence selected from the group consisting of:

   i) a sequence as set forth in SEQ ID NO: 6, 10 or 13;
   ii) a sequence with substitution, deletion or addition of one or several amino acids (e.g., substitution, deletion or addition of one, two, three, four or five amino acids) as compared with the sequence as set forth in SEQ ID NO: 6, 10 or 13; and
   iii) a sequence with a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 6, 10 or 13;

   preferably, the substitution as described in (ii) is a conservative substitution.

4. The use according to any one of claims 1-3, wherein the nucleic acid molecule is a DNA molecule or an RNA molecule; preferably, the nucleic acid molecule is single-stranded or double-stranded.

5. The use according to claim 4, wherein the nucleic acid molecule is an RNA molecule, preferably an mRNA molecule;

   preferably, the nucleic acid molecule comprises a sequence encoding the 3CL protease;
   preferably, the nucleic acid molecule further comprises one or more selected from the group consisting of a 5'UTR, a Kozak sequence, an initiation codon, a termination codon, a 3'UTR, and a poly-A tail;
   preferably, the nucleic acid molecule comprises, in an order from the 5' end to the 3' end, a 5'UTR, a Kozak sequence, an initiation codon, a sequence encoding the 3CL protease, a termination codon, a 3'UTR, and a poly-A tail;
   preferably, the nucleic acid molecule consists of, in an order from the 5' end to the 3' end, a 5'UTR, a Kozak sequence, an initiation codon, a sequence encoding the 3CL protease, a termination codon, a 3'UTR, and a poly-A tail.

6. The use according to claim 5, having one or more characteristics selected from the group consisting of:

   (1) the sequence encoding the 3CL protease is codon-optimized according to the codon bias of a host cell (e.g., a human cell) or non-optimized;
   (2) the sequence encoding the 3CL protease has a sequence with a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 1, 8 or 11; preferably, the sequence encoding the 3CL protease has a sequence as set forth in SEQ ID NO: 1, 8 or 11;
   (3) the nucleic acid molecule carries a 5'modification (e.g., Cap 0, Cap 1, Cap 2) at its 5' end; preferably, the nucleic acid molecule carries a 5'cap 1 modification at its 5' end;
   (4) the nucleic acid molecule comprises one or more N1-methylpseuduridine (m1$\psi$) modifications; preferably, some or all the uracil nucleotide in the nucleic acid molecule is substituted with N1-methylpseudouracil nucleotide;
   (5) the sequence encoding the 3CL protease carries one or more termination codons at its 3' end;
   (6) the 5'UTR has a sequence with a sequence identity of at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 2, preferably, the 5'UTR has a sequence as set forth in SEQ ID NO: 2;
   (7) the 3'UTR has a sequence with a sequence identity of at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 4, preferably, the 3'UTR has a sequence as set forth in SEQ ID NO: 4;

(8) the Kozak sequence has a sequence as set forth in SEQ ID NO: 3;

(9) the poly-A tail comprises one or more polyadenylic acid sequences, the one or more polyadenylic acid sequences each, independently, consisting of 20 to 120 consecutive polyadenylic acid; preferably, the poly-A tail comprises multiple polyadenylic acid sequences and the adjacent polyadenylic acid sequences are linked by a spacer sequence comprising non-A; preferably, the poly-A has a sequence as set forth in SEQ ID NO: 5;

(10) the nucleic acid molecule has a sequence with a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% as compared with the sequence as set forth in SEQ ID NO: 7, 9 or 12; preferably, the nucleic acid molecule has a sequence as set forth in SEQ ID NO: 7, 9 or 12.

7. The use according to any one of claims 1-6, wherein the tumor is a solid tumor or a hematological tumor (such as leukemia, lymphoma, and myeloma);

preferably, the tumor is a solid tumor;
preferably, the tumor is selected from the group consisting of pancreatic cancer, gastric cancer, liver cancer, melanoma, colorectal cancer, renal cancer, cervical cancer, osteosarcoma, prostatic cancer, glioma, and lung cancer;
preferably, types of the pancreatic cancer include, but are not limited to, pancreatic acinar cell carcinoma, adenosquamous carcinoma, squamous cell carcinoma, signet-ring cell carcinoma, undifferentiated carcinoma, undifferentiated carcinoma with giant cells, ampullary carcinoma, pancreatic neuroendocrine tumor, and the like; types of the gastric cancer include, but are not limited to, cardiac cancer, gastric body cancer, and pylorus cancer; types of the liver cancer include, but are not limited to, hepatocellular carcinoma, hepatocellular liver cancer, fibrolamellar liver cancer, intrahepatic cholangiocarcinoma, angiosarcoma, hepatoblastoma, secondary liver cancer, and benign liver tumor; types of the melanoma include, but are not limited to, localized melanoma, regional melanoma, and distant metastatic melanoma; types of the colorectal cancer include, but are not limited to, colon cancer and rectal cancer, specifically including adenocarcinoma, undifferentiated carcinoma, adenosquamous carcinoma, squamous cell carcinoma, small cell carcinoma, carcinoid, mucinous carcinoma, and the like; types of the renal cancer include, but are not limited to, clear cell carcinoma, papillary cell carcinoma, chromophobe cell carcinoma, multilocular cystic renal cell neoplasm of low malignant potential, collecting duct carcinoma, and renal medullary carcinoma; types of the cervical cancer include, but are not limited to, squamous carcinoma, adenocarcinoma, and adenosquamous carcinoma; types of the osteosarcoma include, but are not limited to, telangiectatic osteosarcoma, small round cell osteosarcoma, fibrohistiocytic osteosarcoma, intramedullary well-differentiated osteosarcoma, multicentric osteosarcoma, intracortical osteosarcoma, parosteal osteosarcoma, dedifferentiated parosteal osteosarcoma, periosteal osteosarcoma, and high-grade surface osteosarcoma; types of the prostatic cancer include, but are not limited to, adenocarcinoma (acinar adenocarcinoma), ductal adenocarcinoma, urothelial carcinoma, squamous cell carcinoma, and adenosquamous carcinoma; types of the glioma include, but are not limited to, PAs, astrocytoma, oligoastrocytoma, anaplastic astrocytoma, anaplastic oligoastrocytoma, and glioblastoma; types of the lung cancer include, but are not limited to, small cell lung cancer and non-small cell lung cancer (adenocarcinoma, squamous cell carcinoma, large cell carcinoma, adenosquamous carcinoma, carcinoid, and the like).

8. An isolated nucleic acid molecule, comprising a nucleotide sequence encoding a 3CL protease, wherein the 3CL protease is as defined in any one of claims 1-3;
preferably, the isolated nucleic acid molecule is the nucleic acid molecule as defined in any one of claims 4-6, or the isolated nucleic acid molecule encodes the 3CL protease as defined in any one of claims 1-3.

9. A vector, comprising the isolated nucleic acid molecule of claim 8.

10. A delivery composition, comprising a delivery vehicle, and one or more selected from the group consisting of: a 3CL protease, the isolated nucleic acid molecule according to claim 8, and the vector according to claim 9, wherein the 3CL protease is as defined in any one of claims 1-3;

preferably, the delivery vehicle is used for encapsulating, carrying, or delivering the 3CL protease, the isolated nucleic acid molecule, or the vector;

preferably, the delivery vehicle comprises a non-viral vector, a viral vector, and a virus-like particle (VLP) vector between viral vector and non-viral vector;

preferably, the non-viral vector includes, but is not limited to, cationic liposome, lipid nanoparticle, lipid polymer, artificial microsphere, micelle, lipid-polymer hybrid system, extracellular vesicle, natural or engineered exosome, and the like; the viral vector includes, but is not limited to, adeno-associated virus, lentivirus, adenovirus, retrovirus, vaccinia virus, measles virus, herpes simplex virus, alphavirus, vesicular stomatitis virus, influenza virus, and the like;

preferably, the delivery vehicle is a lipid nanoparticle.

11. A pharmaceutical composition, comprising a 3CL protease, the isolated nucleic acid molecule according to claim 8, the vector according to claim 9 and/or the delivery composition according to claim 10, and a pharmaceutically acceptable carrier and/or excipient, wherein the 3CL protease is as defined in any one of claims 1-3;

preferably, the pharmaceutical composition further comprises an additional anti-tumor agent; preferably, the additional anti-tumor agent is selected from the group consisting of small-molecule chemical drug, biomacromolecule antibody or protein, chimeric antigen receptor cell-based drug, and antibody-drug conjugate;

preferably, the pharmaceutical composition is administered orally, by spray inhalation, rectally, nasally, buccally, vaginally, topically, parenterally such as by subcutaneous, intravenous, intramuscular, intraperitoneal, intrathoracic, intrathecal, intraventricular, intrasternal and intracranial injection or infusion, intratracheal installation, surgical implantation, transdermal delivery, local injection, bolus injection/bombardment, or via an explanted reservoir;

preferably, the medicament is administered orally, intraperitoneally, intrathoracically, or intravenously

12. A method of inhibiting a tumor cell *in vivo* or *in vitro,* comprising: contacting a 3CL protease, the isolated nucleic acid molecule according to claim 8, the vector according to claim 9, the delivery composition according to claim 10 or the pharmaceutical composition according to claim 11 with the tumor cell or delivery them to the tumor cell, wherein the 3CL protease is as defined in any one of claims 1-3;

preferably, the tumor cells are cells of a solid tumor or a hematological tumor (such as leukemia, lymphoma, and myeloma);

preferably, the tumor cell is selected from the group consisting of pancreatic cancer cell, gastric cancer cell, liver cancer cell, melanoma cell, colorectal cancer cell, renal cancer cell, cervical cancer cell, osteosarcoma cell, prostatic cancer cell, glioma cell, and lung cancer cell;

preferably, types of the pancreatic cancer include, but are not limited to, pancreatic acinar cell carcinoma, adenosquamous carcinoma, squamous cell carcinoma, signet-ring cell carcinoma, undifferentiated carcinoma, undifferentiated carcinoma with giant cells, ampullary carcinoma, pancreatic neuroendocrine tumor, and the like; types of the gastric cancer include, but are not limited to, cardiac cancer, gastric body cancer, and pylorus cancer; types of the liver cancer include, but are not limited to, hepatocellular carcinoma, hepatocellular liver cancer, fibrolamellar liver cancer, intrahepatic cholangiocarcinoma, angiosarcoma, hepatoblastoma, secondary liver cancer, and benign liver tumor; types of the melanoma include, but are not limited to, localized melanoma, regional melanoma, and distant metastatic melanoma; types of the colorectal cancer include, but are not limited to, colon cancer and rectal cancer, specifically including adenocarcinoma, undifferentiated carcinoma, adenosquamous carcinoma, squamous cell carcinoma, small cell carcinoma, carcinoid, mucinous carcinoma, and the like; types of the renal cancer include, but are not limited to, clear cell carcinoma, papillary cell carcinoma, chromophobe cell carcinoma, multilocular cystic renal cell neoplasm of low malignant potential, collecting duct carcinoma, and renal medullary carcinoma; types of the cervical cancer include, but are not limited to, squamous carcinoma, adenocarcinoma, and adenosquamous carcinoma; types of the osteosarcoma include, but are not limited to, telangiectatic osteosarcoma, small round cell osteosarcoma, fibrohistiocytic osteosarcoma, intramedullary well-differentiated osteosarcoma, multicentric osteosarcoma, intracortical osteosarcoma, parosteal osteosarcoma, dedifferentiated parosteal osteosarcoma, periosteal osteosarcoma, and high-grade surface osteosarcoma; types of the prostatic cancer include, but are not limited to, adenocarcinoma (acinar adenocarcinoma), ductal adenocarcinoma, urothelial carcinoma, squamous cell carcinoma, and adenosquamous carcinoma; types of the glioma include, but are not limited to, PAs, astrocytoma, oligoastrocytoma, anaplastic astrocytoma, anaplastic oligoastrocytoma, and glioblastoma; types of the lung cancer include, but are not limited to, small cell lung cancer and non-small cell lung cancer (adenocarcinoma, squamous cell carcinoma, large cell carcinoma, adenosquamous carcinoma, carcinoid, and the like).

13. Use of the isolated nucleic acid molecule according to claim 9, the vector according to claim 10, the delivery composition according to claim 11 or 12 or the pharmaceutical composition according to claim 13 in the preparation of a medicament for the prevention and/or treatment of a tumor.

**Fig. 1**

## INTERNATIONAL SEARCH REPORT

| International application No. |
|---|
| **PCT/CN2024/116797** |

**A. CLASSIFICATION OF SUBJECT MATTER**

A61K38/48(2006.01)i;  A61P35/00(2006.01)i

According to International Patent Classification (IPC) or to both national classification and IPC

**B. FIELDS SEARCHED**

Minimum documentation searched (classification system followed by classification symbols)

IPC: A61K;  A61P

Documentation searched other than minimum documentation to the extent that such documents are included in the fields searched

Electronic data base consulted during the international search (name of data base and, where practicable, search terms used)

CNTXT, ENTXTC, CJFD, ENTXT, WFB, WFCCP, WFCD, WFCJ, WFCOA, WFCSTA, WFFCP, WFFD, WFFJ, WFFOA, WFS, WFSTR, PubMed, ISI Web of Knowledge, Baidu, CNKI, GenBank, 中国专利生物序列检索, China Patent Biological Sequence Search: 3CL, 3c-like, 3CL蛋白酶, 3c样蛋白, 冠状病毒, 肿瘤, 癌, cancer, tumor, SARS, MERS, SEQ ID NOs: 6, 10 and 13

**C. DOCUMENTS CONSIDERED TO BE RELEVANT**

| Category* | Citation of document, with indication, where appropriate, of the relevant passages | Relevant to claim No. |
|---|---|---|
| A | CN 113699212 A (INSTITUTE OF MEDICINAL BIOTECHNOLOGY, CHINESE ACADEMY OF MEDICAL SCIENCES) 26 November 2021 (2021-11-26) description, paragraph 3, and sequence listing, SEQ ID NO: 2 | 1-13 |
| A | US 2006142383 A1 (SHANGHAI INSTITUTE OF MATERIA MEDICA, CAS. et al.) 29 June 2006 (2006-06-29) entire document | 1-13 |
| A | CN 115820693 A (XIXIAN NEW AREA FENGHOU ORIGINAL MEDICINE TECHNOLOGY CO., LTD.) 21 March 2023 (2023-03-21) entire document | 1-13 |
| A | CN 115677832 A (HEFEI ZHONGKE PRECEDO BIOMEDICAL TECHNOLOGY CO., LTD.) 03 February 2023 (2023-02-03) entire document | 1-13 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **05 December 2024** | **23 December 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/116797** |

### C. DOCUMENTS CONSIDERED TO BE RELEVANT

| Category* | Citation of document, with indication, where appropriate, of the relevant passages | Relevant to claim No. |
|---|---|---|
| A | CN 1468961 A (SHANGHAI INSTITUTE OF MATERIA MEDICA, CHINESE ACADEMY OF SCIENCES et al.) 21 January 2004 (2004-01-21)<br>entire document | 1-13 |
| A | 马春玲等 (MA, Chunling et al.). "SARS冠状病毒基因组编码蛋白的研究进展 (Non-official translation: Progress in Research on Protein Encoded by SARS Coronavirus Genome)"<br>国外医学病毒学分册 (Section of Virology Foreign Medical Sciences),<br>Vol. 11, No. (1), 29 February 2004 (2004-02-29),<br>pp. 16-19 | 1-13 |

Form PCT/ISA/210 (second sheet) (July 2022)

**INTERNATIONAL SEARCH REPORT**

International application No.

**PCT/CN2024/116797**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/CN2024/116797**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **12**
   because they relate to subject matter not required to be searched by this Authority, namely:

   Claim 12 relates to a method for inhibiting tumor cells in vivo or in vitro, and the technical solution of in-vivo inhibition does not comply with PCT Rule 39.1(iv). A search is carried out on the basis of the corresponding pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/116797**

| Patent document cited in search report | | | Publication date (day/month/year) | Patent family member(s) | | | Publication date (day/month/year) |
|---|---|---|---|---|---|---|---|
| CN | 113699212 | A | 26 November 2021 | None | | | |
| US | 2006142383 | A1 | 29 June 2006 | US | 7662860 | B2 | 16 February 2010 |
| | | | | AU | 2003304184 | A1 | 04 January 2005 |
| | | | | WO | 2004108914 | A1 | 16 December 2004 |
| CN | 115820693 | A | 21 March 2023 | None | | | |
| CN | 115677832 | A | 03 February 2023 | None | | | |
| CN | 1468961 | A | 21 January 2004 | None | | | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311153380

**Non-patent literature cited in the description**

- **NEEDLEMAN et al.** *J. Mol. Biol.*, 1970, vol. 48, 443-453
- **E. MEYERS** ; **W. MILLER**. *Comput. Appl. Biosci.*, 1988, vol. 4, 11-17
- **NEEDLEMAN** ; **WUNSCH**. *J. Mol. Biol.*, 1970, vol. 48, 444-453
- Immunology-A Synthesis. Sinauer Associates, Sunderland, Mass, 1991
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl. Acad. Sci. USA*, 1997, vol. 94, 412-417
- **REMINGTON**. Pharmaceutical Sciences. Mack Publishing Company, 1995
- Molecular Cloning: A Laboratory Manual. Cold Spring Harbor Laboratory Press, 1989
- **J. SAMBROOK** ; **F. M. AUSUBEL et al.** Short Protocols in Molecular Biology. John Wiley & Sons, Inc., 1995